# EUROPÄISCHE PATENTSCHRIFT

(11) **EP 4 447 873 B1**
(45) Veröffentlichungstag und Bekanntmachung des Hinweises auf die Patenterteilung: **25.11.2026**
(21) Anmeldenummer: 22836057.4
(22) Anmeldetag: 12.12.2022
(51) Int. Cl.: A61F 2/60, A61F 2/66, A61F 2/70, A61F 5/01

(54) **ORTHOPÄDIETECHNISCHE EINRICHTUNG UND VERFAHREN ZU DEREN STEUERUNG**
ORTHOPAEDIC DEVICE AND METHOD FOR CONTROLLING IT
DISPOSITIF ORTHOPÉDIQUE ET PROCÉDÉ DE COMMANDE DE CELUI-CI

(30) Priorität: 13.12.2021 DE 102021006127
(43) Veröffentlichungstag der Anmeldung: 23.10.2024
(73) Patentinhaber: Otto Bock Healthcare Products GmbH, 1110 Wien (AT)
(72) Erfinder: SEIFERT, Dirk, 1110 Wien (AT)
(74) Vertreter: Gramm, Lins & Partner Patent- und Rechtsanwälte PartGmbB
(86) Internationale Anmeldenummer: PCT/EP2022/085368
(87) Internationale Veröffentlichungsnummer: WO 2023/110741

(56) Entgegenhaltungen:
- AU-A1- 2015 201 652
- US-A1- 2016 270 997
- US-A1- 2021 069 546
- US-A1- 2021 369 533

## Beschreibung

Die Erfindung betrifft ein Verfahren zur Steuerung einer orthopädietechnischen Einrichtung der unteren Extremität mit einem Oberteil und einem Unterteil, die gelenkig um zumindest eine Schwenkachse miteinander unter Ausbildung eines Gelenkes aneinander gelagert sind und mit zumindest einem Aktuator, der mit einer Steuerungseinrichtung gekoppelt ist, die auf der Grundlage von Sensordaten zumindest eines mit der Steuerungseinrichtung gekoppelten Sensors den Aktuator aktiviert oder deaktiviert, um einen Verschwenkungswiderstand und/oder eine Relativbewegung des Oberteils zu dem Unterteil zu beeinflussen und eine solche orthopädietechnische Einrichtung, insbesondere zur Durchführung des Verfahrens.

Unter orthopädietechnischen Einrichtungen der unteren Extremität werden insbesondere Orthesen und Prothesen verstanden. Orthesen sind orthopädietechnische Hilfsmittel, die an einer existierenden Gliedmaße angelegt werden und die Bewegungen führen, einschränken oder unterstützen. Zwischen gelenkig miteinander verbundenen Komponenten können Antriebe, Aktuatoren und/oder Widerstandseinrichtungen, die über einen Aktuator verstellt oder eingestellt werden können, angeordnet sein. Die Verstellung kann auf Basis von Sensordaten, die einer Datenverarbeitungseinrichtung übermittelt werden, erfolgen. Unter Orthesen werden im Rahmen dieser Anmeldung auch Exoskelette verstanden, die an dem Körper eines Patienten angelegt werden und eine äußere Stützstruktur bilden, insbesondere um die Bewegungen eines Nutzers zu führen und zu beeinflussen, z.B. durch Antriebe zu unterstützen oder über Widerstandseinrichtungen zu bremsen. Orthesen und Exoskelette als deren Spezialfälle können neben der Unterstützung der täglichen Verrichtungen auch zu Trainingszwecken oder zu therapeutischen Zwecken verwendet und eingesetzt werden.

Prothesen ersetzen nicht oder nicht mehr vorhandene Gliedmaßen. Die einfachsten Prothesenkomponenten haben eine rein kosmetische Funktion oder vervollständigen eine Gliedmaße, beispielsweise indem ein distales Fingerglied ersetzt wird. Im Laufe der Zeit wurden die Prothesen komplexer, mehrere Prothesenkomponenten wurden aneinander angeordnet und befestigt und beispielsweise über Gelenke miteinander verbunden. Komplexe mechanische Antriebsvorrichtungen wurden entwickelt, um beispielsweise Prothesenhände oder Prothesenfüße zu bewegen. Hydraulische oder andere Dämpfereinrichtungen oder Widerstandseinrichtungen wurden an Gelenken angeordnet, um das Verhalten von Prothesenkomponenten und Prothesensystemen zu modifizieren, um einen möglichst natürlichen Bewegungsablauf zu ermöglichen. Zur Unterstützung von Bewegungen wurden Antriebe in Prothesenkomponenten integriert, sodass aktive Prothesen entstanden sind. Weiterhin wurden Sensoren an Prothesenkomponenten oder einem Prothesennutzer angeordnet, um das gegenwärtige Bewegungsverhalten oder die gegenwärtigen Positionen oder Stellungen von Prothesenkomponenten zueinander zu erfassen und das zukünftige Bewegungsverhalten abzuschätzen und um Einstellungen an Widerstandseinrichtungen und/oder Antrieben zu verändern. Dadurch sind hochkomplexe Prothesensysteme mit mehreren, aneinander angeordneten Prothesenkomponenten entstanden, die eine Vielzahl von mechanischen, elektrischen und mechatronischen Komponenten aufweisen.

Ein Prothesensystem der unteren Extremität kann insbesondere einen Oberschenkelschaft aufweisen, an dem an dessen distalen Ende ein Prothesenkniegelenk, ein Prothesen-Unterschenkel und ein Prothesenfuß befestigt sind. Ein solches Prothesensystem weist beispielsweise zwei oder mehr Gelenke auf, die jeweils mit Widerstandseinrichtungen und/oder Antrieben bzw. Aktuatoren versehen sein können.

Aus der EP 2 816 979 B1 ist ein Verfahren zur Steuerung eines künstlichen Orthesenkniegelenkes oder Prothesenkniegelenkes bekannt, bei dem auf der Grundlage der Erfassung eines Absolutwinkels einer Unterschenkelkomponente der Beugewiderstand verändert wird. Der ermittelte Absolutwinkel der Unterschenkelkomponente wird mit einem Schwellwert verglichen, wird der Schwellenwert erreicht oder überschritten, wird der Beugewiderstand verändert.

Aus der EP 2 649 968 B1 ist ein Verfahren zur Steuerung eines orthopädischen Fußteils mit einem Knöchelgelenk bekannt, bei dem am Knöchelgelenk auftretende Drehmomente, der Knöchelwinkel sowie der Absolutwinkel eines Fußteils bezogen auf die Vertikale bestimmt werden. In Abhängigkeit von den gemessenen Werten wird das Abrollen des Fußes in der Standphase, die Stellung des Fußteils in der Schwungphase sowie die Stellung und Bewegbarkeit des Fußteils während des Entstehens mittels einer Dämpfungsanordnung gesteuert.

Zur Umschaltung zwischen verschiedenen Betriebsmodi ist es zudem bekannt, dass orthopädietechnische Einrichtungen der unteren Extremität besonders belastet werden, um einen Sondermodus einzustellen. Ein mehrfaches, rhythmisches Belasten in einem bestimmten Zeitraum in einer bestimmten Belastungsrichtung wird als Schaltsignal gewertet, um dann, beispielsweise für das Treppen aufwärts Gehen, besondere Programme zu aktivieren.

Die US 2016/270997 A1 zeigt ein Exoskelett mit einem Hüftrahmen, einem oberen Beinelement, einem unteren Beinelement und einem Fußelement. Der Hüftrahmen und das obere Beinelement sind über ein Hüftgelenk miteinander verbunden. Ein Kniegelenk ist zwischen dem oberen Beinelement und dem unteren Beinelement angeordnet. Ein Knöchelgelenk verbindet das untere Beinelement mit dem Fußelelement. Jedes Gelenk ermöglicht Rotationen um eine erste Achse und eine zweite Achse. Die erste Achse ermöglicht eine Rotation um eine Achse senkrecht zu der Sagittalebene. Die zweite Achse ermöglicht eine Rotation um eine Achse senkrecht zu der Koronalebene. Jeder Achse ist ein Aktuator zum Bewegen von Komponenten um die jeweilige Achse zugeordnet. An der Unterseite des Fußelements sind Druck- und/oder Tastsensoren angeordnet. Die Aktuatoren weisen zudem Sensoren zum Erfassen der Position der Aktuatoren auf. Die Aktuatoren werden auf der Grundlage der Sensordaten angesteuert.

Die AU 2015 201 652 A1 beschreibt ein Exoskelett mit einem Hüftgelenk, das Rotationen in der anterior-posterior-Ebene und in der medial-lateral-Ebene zulässt. Die Rotationen werden mithilfe eines Aktuators ausgeführt, der durch ein Steuergerät angesteuert wird. Es sind ein Beschleunigungssensor und ein Neigungssensor für einzelne Komponenten des Exoskeletts vorgesehen. Die Position und Geschwindigkeit der Aktuatoren wird mithilfe von Positionssensoren erfasst. Weiterhin umfasst das Steuergerät ein Gyroskop für eine Positionssteuerung.

Die US 2021/069546 A1 zeigt ein Übungssystem zur Verbesserung der Leistung der unteren Extremitäten, das die externen Kräfte, die während des Gehens erfahren werden, an einem liegenden Benutzer nachbildet. Der Benutzer bewegt seine Füße in verschiedenen funktionellen Bewegungsmustern und steuert externe Kräfte durch das Aufbringen einer plantaren Kraft auf eine Fußplatte. Mithilfe eines Elektromotors und eines Getriebes wird die Position der Fußplatte senkrecht zur Längsachse des Benutzers gehalten. Die motorisierte Fußplatte passt sich an die medialen/lateralen Bewegungen infolge von Abduktionen/Adduktionen der Hüfte an. Ein integriertes elektronisches System steuert alle Funktionen des Übungssystems. Informationen von Trägheits- Kraft- und Positionssensoren werden an das elektronische System gesendet.

Die US 2021/369533 A1 beschreibt ein Exoskelett zum Stabilisieren und Unterstützen eines Menschen beim Gehen mit zwei Hüftgelenken. Ein Messsystem erfasst einen kombinierten Masseschwerpunkt des Menschen und des Exoskeletts sowie eine Schrittbreite in der Frontalebene. Der Masseschwerpunkt wird insbesondere in Abhängigkeit von einer Beschleunigung, einer Geschwindigkeit und einer Orientierung des Menschen in der Frontalebene bestimmt. Auf der Grundlage des Masseschwerpunkts und der Schrittbreite bestimmt eine Steuerung Einstellwinkel für die Hüftgelenke. Die Hüftgelenke werden auf der Grundlage der Einstellwinkel rotiert.

Das bewusste Umschalten der Betriebsmodi erfordert eine hohe Aufmerksamkeit durch den Nutzer der orthopädietechnischen Einrichtung.

Aufgabe der vorliegenden Erfindung ist es, ein Verfahren und eine orthopädietechnische Einrichtung bereitzustellen, mit denen Nutzern von orthopädietechnischen Einrichtungen der unteren Extremität Aktivitäten des täglichen Lebens einfacher ausführen können und mit denen es möglich ist, eine vielseitigere Nutzung der orthopädietechnischen Einrichtung durchzuführen.

Erfindungsgemäß wird diese Aufgabe durch ein Verfahren mit den Merkmalen des Hauptanspruches gelöst und eine orthopädietechnische Einrichtung mit den Merkmalen des nebengeordneten Anspruchs gelöst. Vorteilhafte Ausgestaltungen und Weiterbildungen der Erfindung sind in den Unteransprüchen, der Beschreibung sowie den Figuren offenbart.

Das Verfahren zur Steuerung einer orthopädietechnischen Einrichtung der unteren Extremität mit einem Oberteil und einem Unterteil, die gelenkig um zumindest eine Schwenkachse miteinander unter Ausbildung eines Gelenkes aneinander gelagert sind und mit zumindest einem Aktuator, der mit einer Steuerungseinrichtung gekoppelt ist, die auf der Grundlage von Sensordaten zumindest eines mit der Steuerungseinrichtung gekoppelten Sensors den Aktuator aktiviert oder deaktiviert, um einen Verschwenkungswiderstand und/oder eine Relativbewegung des Oberteils zu dem Unterteil zu beeinflussen, sieht vor, dass über die Sensordaten eine Orientierung, Verlagerung oder eine Kombination aus Orientierung und/oder Verlagerung der orthopädietechnischen Einrichtung in der Frontalebene erfasst wird und dass auf der Grundlage der Orientierung und/oder Verlagerung in der Frontalebene der Aktuator aktiviert, deaktiviert und/oder ein Sollwert für den Aktuator moduliert wird. Während in dem Stand der Technik ausschließlich die Bewegungen in der Sagittalebene analysiert, ausgewertet und zur Modifikation der Widerstände oder Antriebe eingesetzt werden, wird erfindungsgemäß eine Seitwärtsneigung, eine Seitwärtsrotation und/oder eine Seitwärtsbewegung in der Frontalebene eines Nutzers erfasst und zur Steuerung von Widerständen und/oder Antrieben eingesetzt, insbesondere zur Steuerung von Bewegungswiderständen und/oder Bewegungen. Die Einbeziehung der Sensordaten hinsichtlich der Orientierung und/oder Verlagerung der orthopädietechnischen Einrichtung innerhalb der Frontalebene, in der Regel quer zu der üblichen Vorwärtsbewegung, kann auch in Ergänzung mit anderen Steuerungsverfahren genutzt werden. Dabei werden Orientierungen und/oder Verlagerungen innerhalb der Frontalebene von einzelnen Komponenten der orthopädietechnischen Einrichtung oder auch von mehreren oder allen Komponenten der orthopädietechnischen Einrichtung erfasst. Insbesondere bei Mehrgelenkeinrichtungen wie Orthesen oder Prothesen, die sowohl das Knöchelgelenk als auch das Kniegelenk umfassen, sind die Gelenkachsen der orthopädietechnischen Einrichtungen regelmäßig parallel oder annähernd parallel zueinander orientiert und befinden sich in der Frontalebene, wenn die Gelenkachsen übereinander stehen. Die Gelenkigkeit zwischen einem Oberteil und einem Unterteil kann auch durch mehrere Achsen ausgebildet sein. Derartige Gelenke werden als polyzentrische Gelenke bezeichnet. Orthopädietechnische Einrichtungen können auch das Hüftgelenk übergreifen. Ein solches Hüftgelenk kann so ausgebildet sein, dass es sich ebenfalls um eine Achse verschwenken lässt, welche im Wesentlichen in der Frontalebene liegt. Es ist jedoch auch möglich, dass eine Verschwenkung um weitere Achsen möglich ist, bspw. eine Abduktionsbewegung um eine Achse normal auf die Frontalebene. Neben der Verschwenkung um Achsen ist insbesondere für das Hüftgelenk eine ergänzende oder alternative Verschieblichkeit und/oder eine kugelige Lagerung vorteilhaft. **In** der Regel sind die Gelenkachsen in der Frontalebene in der Ausgangsposition dabei waagerecht orientiert. **In** der Ausgangsposition finden sich die Oberteile und Unterteile in einer im Wesentlichen senkrechten Orientierung, sodass die Längserstreckung der orthopädietechnischen Einrichtung im Wesentlichen der Vertikalen entspricht. Sobald die orthopädietechnische Einrichtung in der Frontalebene verschwenkt wird, ändert sich die Orientierung der Längserstreckung und somit auch die Orientierung der Schwenkachsen. Die Verschwenkung erfolgt dabei in der Standphase bei einem aufgesetzten Fuß um einen Schwenkpunkt oder eine Schwenkachse an dem distalen Ende oder in dem distalen Endbereich der orthopädietechnischen Einrichtung. Der Fuß oder das distale Ende bleibt relativ zu dem Untergrund stationär, der Körper des Nutzers der orthopädietechnischen Einrichtung wird bewegt. Ist beispielsweise das rechte Bein mit der orthopädietechnischen Einrichtung versorgt, erfolgt eine Verschwenkung in der Frontalebene in der Standphase überwiegend dadurch, dass das linke Bein von der orthopädietechnischen Einrichtung weg nach links bewegt wird. Eher selten findet ein Übersetzen des linken Fußes auf die rechte Seite des versorgten Beines statt, wobei dies ebenfalls eine Bewegung in der Frontalebene ist. Eine Verschwenkung und/oder Bewegung in der Frontalebene kann auch mit einer Verschwenkung, Rotation und/oder Bewegung in einer anderen Ebene kombiniert sein, beispielsweise wenn das Bein über einen distalen Abrollpunkt nach diagonal vorne verschwenkt wird. Die Bewegung findet damit teilweise in der Frontalebene statt, jedoch nicht zwangsweise ausschließlich. Die Steuerung kann sich auf eine Bewegungskomponente beziehen, insbesondere eine Bewegungskomponente in der Frontalebene. **In** Abhängigkeit von der Neigung oder Verschwenkung der orthopädietechnischen Einrichtung in der Frontalebene wird der maximale Verschwenkwinkel des Oberteils zu dem Unterteil verstellt. Je größer die Verschwenkung der gesamten orthopädietechnischen Einrichtung oder auch nur einer Komponente davon innerhalb der Frontalebene ist, desto größer kann der Verschwenkwinkel des Oberteils zu dem Unterteil eingestellt werden, sodass beispielsweise eine größere Knieflexion ermöglicht wird. Die Änderung in den maximalen Verschwenkwinkeln kann stufenweise oder kontinuierlich erfolgen.

In der Schwungphase der beispielsweise versorgten rechten Seite bleibt das beispielsweise unversorgte Bein auf dem Boden und das versorgte rechte Bein wird angehoben und nach rechts abgespreizt, beispielsweise um den gesamten Körper nach rechts zu verlagern. Auch hier ist ein Übersetzen auf die linke Seite des linken, unversorgten Beines möglich und führt zu einer Verschwenkung oder Verlagerung in der Frontalebene. Solche Bewegungen oder Zustände in der Frontalebene werden erfasst und dienen als Grundlage dafür, ob ein Aktuator aktiviert oder deaktiviert oder ein Sollwert für den Aktuator moduliert oder geändert wird, beispielsweise um einen Verschwenkungswiderstand eines Oberteils zu einem Unterteil über Widerstandseinrichtung und/oder eine Relativverlagerung von Oberteil zu Unterteil über einen Antrieb, beispielsweise einen Motor, oder eine Freigabe gespeicherter Energie zu beeinflussen. Es können auch mehrere Aktuatoren angesteuert werden. Findet keine Bewegung in der Frontalebene statt, sondern ausschließlich beispielsweise in der Sagittalebene, ändert sich die Ansteuerung des mindestens einen Aktuators und es greifen andere Steuerungsmechanismen und/oder ein anderes Steuerungsprogramm wird aktiviert, um über den Aktuator den Verschwenkungswiderstand und/oder eine Relativbewegung des Oberteils zu dem Unterteil zu beeinflussen. Das erfindungsgemäße Verfahren ist insbesondere ein Teil einer komplexen Steuerung der orthopädietechnischen Einrichtung, über die Widerstände gegen eine Verschwenkung beeinflusst oder ein Antrieb auf der Grundlage einer Vielzahl von Sensordaten aktiviert, deaktiviert oder ein Sollwert für den Aktuator moduliert wird.

In einer Weiterbildung des Verfahrens werden mehrere Freiheitsgrade der orthopädietechnischen Einrichtung gesteuert. Hierbei kann es sich sowohl um einen oder mehrere zusätzliche Freiheitsgrade eines Gelenks handelt. Alternativ oder ergänzend können auch mehrere Gelenke und/oder Komponenten gesteuert werden. Eine orthopädietechnische Einrichtung kann beispielsweise Knöchel und Knie, Knie und Hüfte oder Knöchel, Knie und Hüfte umgreifen. Ein Gelenk kann zudem mehrere Freiheitsgrade aufweisen, bspw. ein Hüftgelenk, das sowohl eine Flexion-Extension zulässt als auch eine Adduktion-Abduktion. Es müssen jedoch nicht zwangsweise alle Freiheitsgrade einer orthopädietechnischen Einrichtung gesteuert werden.

In einer Weiterbildung des Verfahrens ist es vorgesehen, dass während der Nutzung der orthopädietechnischen Einrichtung im angelegten Zustand die Sensordaten ermittelt werden und dass der Aktuator aktiviert, deaktiviert oder ein Sollwert für den Aktuator moduliert wird, um den Verschwenkungswiderstand und/oder eine Relativbewegung des Oberteils zu dem Unterteil zu beeinflussen.

Die Orientierung und/oder die Verlagerung der orthopädietechnischen Einrichtung oder Teilen der orthopädietechnischen Einrichtung wird in einer Ausgestaltung über einen Raumlagesensor, zumindest eine IMU (inertial measurement unit) und/oder zumindest einen Winkelsensor erfasst und ermittelt. Die Winkelsensoren oder der Winkelsensor erfasst die Stellung von Oberteil zu Unterteil, einzelner Komponenten zueinander oder auch der Komponenten relativ zu einem Körperteil oder einem anderen Bezugselement an dem Nutzer und ermöglichen die Feststellung der Orientierung der gesamten orthopädietechnischen Einrichtung, mehrerer Teile davon oder auch nur eines Teils davon innerhalb der jeweiligen Ebene, insbesondere innerhalb der Frontalebene. Über einen Raumlagesensor oder eine IMU können unmittelbar die Orientierungen von Komponenten bzw. der gesamten orthopädietechnischen Einrichtung und sofern notwendig die die Orientierung der Komponenten zueinander bestimmt und nach der Auswertung der Steuerung zur Aktivierung, Deaktivierung oder Modulierung eines Sollwertes des Aktuators zur Veränderung des Verschwenkungswiderstandes und/oder der Relativbewegung entsprechende Befehle übermittelt werden. Ein Relativwinkel zwischen zwei Komponenten kann aus den jeweiligen Absolutwinkeln bestimmt werden, beispielsweise durch zwei IMUs.

In einer Weiterbildung werden Kräfte, Momente und/oder Beschleunigungen der gesamten orthopädietechnischen Einrichtung und/oder deren Komponenten über Sensoren erfasst und der Steuerung zugrunde gelegt. In Verbindung mit der Erfassung von Verschwenkungen und/oder Verlagerungen innerhalb der Frontalebene werden zusätzliche Parameter zur Steuerung des Aktuators herangezogen. Ein Wegfall von Axialkräften zeigt beispielsweise, dass die orthopädietechnische Einrichtung sich in einer Schwungphase befindet. Unterschiedliche Kraftverteilungen oder Einleitungen von Momenten um Schwenkachsen ermöglichen eine Bestimmung von Bewegungen, Bewegungsänderungen, Zuständen und wahrscheinlichen zukünftigen Bewegungen oder Belastungen, sodass auf Grundlage der Kräfte, Momente und/oder Beschleunigungen, insbesondere in Verbindung mit Daten zur Orientierung und/oder Verlagerung innerhalb der Frontalebene der Aktuator mit entsprechenden Befehlen versorgt wird. Kräfte und Momente können über eine Verformung, Verschiebung, Verkippung und/oder eine Kombination aus diesen bestimmt werden. Zum Beispiel kann aus der Verformung eines elastischen oder kompressiblen Körpers und/oder aus einer darauf resultierenden Verkippung oder Verschiebung auf eine einwirkende Kraft und/oder ein Moment geschlossen werden. Über die Verschiebungs- oder Verformungsrate eines Dämpfers oder viskosen Elements kann ebenfalls auf eine Kraft und/oder ein Moment geschlossen werden.

In einer Ausführungsform werden nur einzelne Messgrößen in der Frontalebene ermittelt oder gemessen und damit auf eine Verschwenkung in der Frontalebene geschlossen. Beispielsweise kann ein Moment um den Knöchel lediglich in der Sagittalebene ermittelt werden, während die Beschleunigung sowohl in der Sagittalebene als auch in der Frontalebene bestimmt wird. Es ist auch möglich, dass einzelne Messgrößen ausschließlich in der Frontalebene bestimmt werden. Durch ein minimales Sensor-Set kann eine orthopädietechnische kostengünstig hergestellt und die Robustheit erhöht werden.

In einer Ausgestaltung ist die orthopädietechnische Einrichtung als Prothese oder Orthese ausgebildet und weist ein künstliches Kniegelenk und/oder Knöchelgelenk auf, wobei jedem Gelenk ein Aktuator zugeordnet ist. Sind sowohl ein Kniegelenk als auch ein Knöchelgelenk vorhanden, können zwei Aktuatoren vorhanden sein, um individuell und unabhängig voneinander einen Verschwenkungswiderstand um das jeweilige Gelenk zu verändern bzw. eine Relativbewegung zwischen dem jeweiligen Oberteil und dem jeweiligen Unterteil einzuleiten oder zu beeinflussen. Es besteht auch die Möglichkeit, dass nur ein einziger Aktuator zwei Gelenken zugeordnet ist, über den eine entsprechende Vergrößerung oder Verkleinerung eines Verschwenkungswiderstandes erreicht oder eine Verlagerung von Oberteil zu Unterteil bewirkt wird. Es können auch mehr als zwei Gelenke und ein oder mehrere Aktuatoren angeordnet sein und gesteuert werden.

**In** einer Weiterbildung werden translatorische Verlagerungen der orthopädietechnischen Einrichtung erfasst und der Steuerung ebenfalls zugrunde gelegt. Die Erfassung und Berechnung von translatorischen Bewegungen einer orthopädietechnischen Komponente kann beispielsweise über eine Wegintegration in der jeweiligen Ebene erfolgen und lässt Rückschlüsse auf die Art und den Umfang der Bewegung der orthopädietechnischen Komponente an der jeweiligen Bezugsstelle, beispielsweise in dem distalen Endbereich der orthopädietechnischen Einrichtung oder an einem Gelenk zu.

**In** einer Weiterbildung werden relative Abstände zwischen Komponenten der orthopädietechnischen Einrichtung und/oder zwischen der orthopädietechnischen Einrichtung, Gliedmaßen und/oder Körperteilen der nutzenden Person, der Umgebung und oder anderen orthopädietechnischen Einrichtungen bestimmt. Es können auch Abstände zur Umgebung, insbesondere zum Boden bestimmt werden. Relative Abstände können entlang einer kinematischen Kette beispielsweise durch ein oder mehrere Relativwinkel- und/oder Absolutwinkel-Sensoren unter Zuhilfenahme von Segmentlängen bestimmt werden. Zum Beispiel kann bei bekannter Unter- und Oberschenkellänge aus dem Kniewinkel der Relativabstand von Knöchel und Hüfte berechnet werden. Alternativ oder ergänzend können Abstände direkt gemessen werden. **In** einer Ausführungsform wird zumindest ein Abstand zwischen zwei Punkten über eine Umgebungs-Sensorik bestimmt. Die Umgebungs-Sensorik bestimmt die Anordnung von Objekten zu der orthopädietechnischen Einrichtung und oder zwischen Objekten, zum Beispiel durch Wechselwirkung mit elektromagnetischer Strahlung. Objekte können zum Beispiel durch die von ihnen emittierte, reflektierte oder re-emittierte elektromagnetische Strahlung erfasst werden. Es ist möglich, Objekte durch eine oder mehrere Kameras zu erfassen, die im sichtbaren und nicht sichtbaren Wellenlängenbereich arbeiten. Auch können Tiefenbildkameras verwenden werden. Es können Lidar, Sonar, Radar und/oder ähnliche Sensorik verwendet werden, um die Umgebung zu erfassen. Objektabstände können unter anderem durch Laufzeitmessungen und/oder Triangulation bestimmt werden. Relative Geschwindigkeiten können bspw. über den Dopplereffekt bestimmt werden. Es können mehrere Sensoren und/oder Sensor-Arrays an der orthopädietechnischen Einrichtung angeordnet sein, um aus mehreren Messungen, zum Beispiel mehreren Abstands- und/oder Laufzeitmessungen, die Richtung eines Objektes und/oder die Anordnung, Lage und/oder Orientierung von Objekten in Relation zur orthopädietechnischen Einrichtung zu bestimmen. Bei dem ein oder den mehreren Objekten, respektive der Umgebung kann es sich um andere Komponenten der orthopädietechnischen Einrichtung, Gliedmaße oder Körperteile des eigenen Körpers, den Untergrund und/oder Objekte in der Umgebung der anwendenden Person handeln. Es ist auch möglich, die geometrische Konfiguration durch externe Sensoren zu erfassen, zum Beispiel eine oder mehrere Kameras, und die Information an die Steuerung der orthopädietechnischen Einrichtung weiterzuleiten. Abstände und/oder geometrische Anordnungen können auch über einen oder mehrere Empfänger und Sender, die sich an unterschiedlichen Positionen befinden, ermittelt werden. Abstände und Anordnungen von Komponenten können insbesondere in der Frontalebene bestimmt werden und/oder in der Frontalebene errechnet oder erfasst werden.

In einer Ausgestaltung des Verfahrens wird in der Standphase bei einer Neigung oder Verschwenkung der versorgten Seite mit der orthopädietechnischen Einrichtung in Medialrichtung eine Verringerung eines Flexionswiderstandes oder eine Flexionsbewegung in Abhängigkeit der Neigung, insbesondere bei Erreichen eines Schwellwertes der Neigung, eingeleitet. Während der Standphase findet eine Neigung oder Verschwenkung in Medialrichtung durch eine Verschwenkung oder Verkippung um einen distalen Auflagepunkt oder eine distale Schwenkachse in Richtung auf die Körpermitte relativ zu der Längserstreckung der orthopädietechnischen Einrichtung in der Ausgangsstellung bzw. relativ zu der Vertikalen oder der Schwerkraftlinie statt. In Abhängigkeit der Verschwenkung wird in einer Ausgestaltung der Flexionswiderstand verringert oder bei einem aktiven Antrieb ein Flexionsmoment aufgebracht und/oder eine Flexionsbewegung eingeleitet, insbesondere wenn die Neigung oder der Verschwenkwinkel während einer Bewegung der versorgten Seite in der Standphase einen Schwellwert übersteigt. Für ein Kniegelenk bedeutet dies, dass eine Einbeugung noch in der Standphase erleichtert oder eingeleitet wird, sodass insbesondere bei Orthesen das Knie gebeugt und damit der Fuß leichter angehoben werden kann oder angehoben wird. Auch bei Prothesen werden damit das Seitwärtsgehen und/oder eine Richtungsänderung erleichtert. Auch bei einer Verschwenkung nach lateral ist es möglich, Widerstände und oder Bewegungen anzupassen, beispielsweise in ähnlicher Art und Weise wie bei einer Verschwenkung nach medial.

**In** einer Ausgestaltung wird bei einer Verringerung der Axialbelastung oder in einer Anhebephase der versorgten Seite eine Verringerung des Flexionswiderstandes oder eine Flexion eingeleitet. Durch eine Verringerung der Axialbelastung oder durch die Detektion eine Anhebephase, was nach einer Erkennung oder Detektion der terminalen Standphase durch Auswertung von Sensordaten wie Kraftverteilung, Kraftverlauf, Momentenverteilung, Momentenverlauf und Winkelstellungen von Komponenten zueinander erfolgen kann, wird bereits vorsorglich der Flexionswiderstand verringert oder eine Flexion eingeleitet. Dies ist insbesondere bei künstlichen Kniegelenken wie Prothesenkniegelenken und Orthesenkniegelenken vorteilhaft. Bei Kniegelenken wird der Flexionswiderstand verringert oder eine Flexion aktiv eingeleitet, insbesondere bei Orthesenknöchelgelenken wird beispielsweise der Dorsalflexionswiderstand verringert oder eine Dorsalflexion eingeleitet, insbesondere in der Schwungphase, jeweils um ein Durchschwingen zu erleichtern und eine größere Bodenfreiheit bereitzustellen.

**In** einer Ausgestaltung wird in einem Knöchelgelenk bei einer Verschwenkung in der Frontalebene in der Standphase ein Push-Off erzeugt, bei dem durch eine Plantarflexion ein Impuls nach oben gegeben und/oder ein zu schnelles Absinken des Körperschwerpunkts verhindert wird. Der Push-Off kann unter anderem zeitlich gesteuert oder die Folge einer Kraft-Verschiebungs-Charakteristik sein, zum Beispiel eines elastischen Verhaltens. Die Intensität des Push-Offs, zum Beispiel die Dauer, das Bewegungsausmaß, die Maximalkraft, die Leistung und/oder die verrichtete Arbeit, kann mit der Verschwenkung in der Frontalebene, zum Beispiel der Verschwenkung in der terminalen Standphase, angepasst werden, insbesondere bei einer Verschwenkung reduziert oder erhöht werden.

Eine Ausgestaltung sieht vor, dass eine Verringerung eines Flexionswiderstandes oder eine aktive Flexion nur bei einer Vorwärtsneigung oder Vorwärtsverschwenkung oder bis zu einem gewissen Ausmaß an Rückwärtsneigung oder Rückwärtsverschwenkung der orthopädietechnischen Einrichtung oder einer Komponente davon eingeleitet wird. Eine Vorwärtsverschwenkung ist eine Verschwenkung innerhalb der Sagittalebene nach anterior, die üblicherweise die Gehrichtung eines Nutzers einer orthopädietechnischen Einrichtung ist und typischerweise der Blickrichtung entspricht.

Bei einer Neigung oder Verschwenkung der versorgten Seite in der Standphase in Medialrichtung bei gleichzeitiger Erfassung einer Rückwärtsneigung und/oder einer Rückwärtsverschwenkung und/oder bei Erfassung einer hinreichend kleinen Vorwärtsneigung und/oder Vorwärtsverschwenkung findet in einer Ausgestaltung keine Verringerung oder eine Erhöhung eines Flexionswiderstandes statt. Alternativ oder ergänzend kann auch ein Streckmoment aufgebracht werden. Dadurch wird die orthopädietechnische Einrichtung insgesamt stabiler und ein größeres Maß an Sicherheit wird für den Nutzer der orthopädietechnischen Einrichtung bereitgestellt.

Bei der Neigung oder Verschwenkung der versorgten Seite in der Standphase in Medialrichtung bei gleichzeitiger Erfassung einer Rückwärtsneigung oder Rückwärtsbewegung bzw. Rückwärtsverschwenkung und/oder bei Erfassung einer hinreichend kleinen Vorwärtsneigung und/oder Vorwärtsverschwenkung, wird eine Flexionsbewegung abgestoppt oder eine Extensionsbewegung eingeleitet, sodass durch entsprechende Aktivierung oder Deaktivierung eines Antriebes oder Veränderung eines Sollwertes für den Aktuator eine Relativbewegung zwischen dem Oberteil und dem Unterteil um die Schwenkachse verändert wird. Dies ist insbesondere bei Kniegelenken sinnvoll, um eine verbesserte Standsicherheit zu erreichen.

Bei einer Neigung oder Verschwenkung der mit der orthopädietechnischen Einrichtung versorgten Seite in der Standphase in Medialrichtung kann ein künstliches Knöchelgelenk in Plantarflexionsrichtung angetrieben oder der Dorsalflexionswiderstand erhöht oder nicht verringert werden, um entweder eine Plantarflexion auszuführen oder ein Abdrücken zur Seite zu ermöglichen bzw. zu unterstützen.

Wird in der Standphase bei einer Neigung oder Verschwenkung der versorgten Seite gleichzeitig ein Flexionsmoment festgestellt, beispielsweise ein Moment, das eine Knieflexion bewirkt oder bewirken würde oder eine Dorsalflexion innerhalb des Knöchelgelenkes, ist einer Ausgestaltung vorgesehen, dass keine Verringerung oder eine Erhöhung eines Flexionswiderstandes eingeleitet wird oder ein Extensionsmoment durch einen Antrieb aufgebracht wird. Dies erhöht die Stabilität der orthopädietechnischen Einrichtung und erleichtert das Gehen um Kurven, insbesondere um enge Kurven.

Bei der Verschwenkung der versorgten Seite in einer Anhebephase der Schwungphase in Lateralrichtung, wenn die versorgte Seite von dem Körper weg bewegt wird, findet in einer Ausgestaltung eine Verringerung eines Flexionswiderstandes, insbesondere gegen eine Knieflexion statt. Alternativ oder ergänzend wird eine aktive Knieflexion eingeleitet. Der Dorsalextensionswiderstand eines Fußes oder Fußteils kann reduziert werden und/oder eine Dorsalextension eingeleitet werden.

Eine Extension in einem künstlichen Kniegelenk kann in der Schwungphase, insbesondere bei einer Verschwenkung der versorgten Seite in Lateralrichtung verzögert oder verhindert werden, um eine vollständige Bewegung bis zum Ende durchzuführen, insbesondere um eine entsprechende Bodenfreiheit zu ermöglichen.

Eine Extension kann in einem künstlichen Kniegelenk zeitgesteuert freigegeben oder bewirkt werden. Alternativ oder ergänzend kann in Abhängigkeit des Verschwenkwinkels und/oder der Bewegung, insbesondere nach Erreichen eines Schwellwertes, eine Veränderung der Verschwenkgeschwindigkeit, einer Veränderung der Verschwenkrichtung und/oder auf der Grundlage einer Hüftextensionsbewegung und/oder der Oberschenkelbewegung die Extension in dem künstlichen Kniegelenk freigegeben oder bewirkt werden.

Bei einer hüftübergreifenden orthopädietechnischen Einrichtung kann eine Hüftextension zunächst verhindert oder verlangsamt und zu einem späteren Zeitpunkt freigegeben werden. Ein Freigeben kann auch eine Reduktion der Verlangsamung sein.

Auf der Grundlage der Neigung und/oder Verlagerung in der Frontalebene kann ein Sondermodus der Steuerung aktiviert oder deaktiviert werden.

In einer Ausgestaltung wird ein Bewegungsmodus und/oder eine Sonderfunktion unterbrochen, gewechselt oder verlassen, wenn eine Seitwärtsbewegung und/oder ein Verschwenken in der Frontalebene erkannt werden. Ein Bewegungsmodus kann eine Steuerung sein, welche einen spezifischen Bewegungsablauf steuert, wie zum Beispiel das Hinaufgehen auf eine Treppe, das Hinsetzen oder das Rennen. Eine orthopädietechnische Einrichtung kann fließend von einem Bewegungsmodus in einen anderen wechseln, beispielsweise vom Aufstehen in das Vorwärtsgehen. Für viele Bewegungsabläufe sind eine Seitwärtsbewegung und/oder ein Verschwenken in der Frontalebene untypisch und kann als Indikator dafür herangezogen werden, dass von dem ursprünglichen Bewegungsablauf in einen anderen Bewegungsablauf gewechselt werden und damit die Steuerung oder das zugrundeliegende Steuerungsgesetz angepasst werden soll. Wird zum Beispiel während einer schnellen Hüftflexion, welche zunächst als Bewegungsablauf des Treppe nach oben Gehens angesehen wird und die orthopädietechnische Einrichtung eine entsprechende Steuerung für diesen Bewegungsablauf bereit stellt, eine zusätzliche Seitwärtsbewegung und/oder ein Verschwenken in der Frontalebene erkannt, zum Beispiel eine Abduktion der Hüfte, wird vorzugsweise der Bewegungsablauf des Treppe nach oben Gehens abgebrochen. **In** weiterer Folge wird zum Beispiel in einen Grundzustand geschaltet, welcher anstatt einer aktiven Knieflexion eine teilweise Kniestreckung durchführt. Das Verhalten der orthopädietechnischen Einrichtung wird so angepasst, dass bei einer Verschwenkung in der Frontalebene die Steuerung für einen spezifischen Bewegungsablauf abgebrochen wird und sich das Verhalten ändert. **In** manchen Bewegungsabläufen kann ein Verschwenken in der Frontalebene auch ein Indikator für eine sicherheitskritische Situation sein, welche es notwendig macht, die Steuerungsstrategie zu ändern. Ein Verschwenken in der Frontalebene, insbesondere um eine distale Schwenkachse oder einen Schwenkpunkt, kann zum Beispiel auf einen Gleichgewichtsverlust hindeuten, wenn es sich beim eigentlichen Bewegungsmodus um eine Bewegung handelt, die im Wesentlichen in der Sagittalebene stattfindet. **In** Folge kann die Steuerung angepasst werden. Eine Anpassung der Steuerung kann zum Beispiel eine Veränderung des Bewegungswiderstandes, einer Kraft-Weg-Charakteristik und/oder die Anpassung einer Stellung und/oder Bewegung sein. Es ist möglich, dass ein Bewegungsmodus nur temporär aufgrund einer Verschwenkung in der Frontalebene unterbrochen wird, beispielsweise so lange eine Seitwärtsneigung vorhanden ist. Ist keine Seitwärtsneigung mehr vorhanden, werden der entsprechende Bewegungsmodus sowie die damit verbundene Steuerung wieder ausgeführt.

Bei einer Sonderfunktion kann es sich um eine Funktion handeln, die durch eine spezielle Nutzerinteraktion eingestellt wird. Beispielhaft seien eine Funktion für das Fahrradfahren, ein Sportmodus, eine Sonderfunktion für das Autofahren und eine Standby-Funktion genannt. Eine Sonderfunktion kann durch ein spezifisches Bewegungs- und/oder Belastungsmuster aktiviert werden, zum Beispiel ein Wippmuster auf dem Vorfuß. Alternativ oder ergänzend kann über ein Interface, wie zum Beispiel ein Bedienelement oder eine App, in eine Sonderfunktion geschalten werden. Im Weiteren ist es möglich, dass eine Sonderfunktion unter anderem dann beendet wird, wenn ein Verschwenken in der Frontalebene erkannt wird. Beispielsweise kann eine Fahrradfunktion verlassen werden, wenn eine Seitwärtsbewegung und/oder Seitwärtsverschwenkung erkannt wird, insbesondere eine plötzliche Seitwärtsbewegung des Fußes und/oder ein Abspreizen des Beines. Eine solche Bewegung ist ein Indikator dafür, dass die Person vom Fahrrad absteigen möchte. Es ist daher vorteilhaft die Fahrradfunktion und die damit verbundene Steuerung zu beenden und in einen Modus zu wechseln, welcher besonders für das Stehen geeignet ist.

Wird die orthopädietechnische Einrichtung auf Basis einer Verschwenkung in der Frontalebene gesteuert, ist es möglich, dass ein gewisses Mindestmaß an Bewegung oder Änderung der Bewegung mit der Zeit und deren beliebige Ableitungen notwendig ist, um einen Einfluss auf die Steuerung zu haben. Dies kann über einen oder mehrere Schwellwerte und/oder über komplexere Algorithmen umgesetzt werden, bspw. über einen Mehrheitsentscheid über mehrere Werte oder über künstliche Intelligenz.

Sämtliche Steuerungsalgorithmen, welche ein Verschwenken in der Frontalebene oder daraus abgeleitete Größen als Eingangsgröße aufweisen, können auch weitere Eingangsgrößen haben, insbesondere Bewegungen in andere Richtungen, Belastungen und/oder Informationen von anderen Sensoren, welche Einfluss auf das Verhalten der orthopädietechnischen Einrichtung haben.

In einer Ausführungsform wir die Steuerung auf Basis der Verschwenkung in der Frontalebene über zumindest ein Biosignal kombiniert. Biosignale sind jegliche Signale, welche Informationen über Eigenschaften des Körpers sowie Informationsflüsse im Körper beinhalten. Bei einem Biosignal kann es sich um ein elektrisches Signal handeln, zum Beispiel Nervensignale, elektrische Signale bei Kontraktion der Muskulatur wie EMG oder Elektromyographie, aber auch Gehirnströme. Sind Informationen in einem Signal kodiert, kann zunächst eine Dekodierung erfolgen, bevor das Signal für die Steuerung herangezogen wird. Ein Biosignal kann ein chemisches oder elektrochemisches Signal sein, zum Beispiel die Konzentration eines Stoffes, die Wechselwirkung von Molekülen oder ein elektrochemischer Gradient. Biosignale können auch Leitfähigkeiten von Geweben und/oder Körperteilen sein. Bei Biosignalen kann es sich auch um mechanische Parameter wie Kraft, Druck und Länge sowie deren zeitliche Verändert handeln, beispielsweise der Puls oder Längenänderung eines Muskels. Biosignale können invasiv oder nichtinvasiv erfasst werden. In einer Ausgestaltung wird die Steuerung über zumindest die erfasste Verschwenkung in der Frontalebene durch zumindest ein Biosignal moduliert, insbesondere über ein Biosignal das mit einer physischen oder intendierten Muskelanspannung, Kontraktion und/oder Ansteuerung des Bewegungsapparates in Verbindung steht. Durch die Modulation mittels eines solchen Biosignals ist es der tragenden Person möglich, Einfluss auf die Steuerung und damit die Bewegung und/oder den Bewegungswiderstand zu nehmen. Die Modulation kann ein Wechseln zwischen unterschiedlichen Steuerungs-Charakteristiken sein oder die kontinuierliche Veränderung der Steuerungs-Charakteristik, insbesondere eines Bewegungswiderstandes und/oder einer Bewegung. Die versorgte Person kann so zum Beispiel durch Anspannung der Muskulatur in einer Abhebephase eines Seitwärtsschrittes das Ausmaß an Kniebeugung bestimmen. Bei einem passiven Freiheitsgrad ist es möglich, dessen Bewegungswiderstand in Abhängigkeit der Muskelanspannung zu steuern, zum Beispiel den Bewegungswiderstand bei Anspannung zu erhöhen. Es kann zum Beispiel der Kniewinkel und/oder der Hüftwinkel nach einer Einbeugephase durch Anspannung der Muskulatur und/oder ein anderes Biosignal konstant gehalten und eine Streckung mit der Schwerkraft verhindert werden. Alternativ kann die Streckbewegung verlangsamt werden. Bei Nachlassen der Muskelkontraktion wird die Bewegung wieder zugelassen bzw. der Streckwiderstand reduziert. Alternativ oder ergänzend kann ein Verschwenken in der Frontalebene einen Einfluss auf die Signalverarbeitung eines Biosignals haben. Zum Beispiel kann ein Biosignal in Abhängigkeit der Seitwärtsbewegung und/oder Neigung einen unterschiedlichen Einfluss auf die Steuerung der orthopädietechnischen Einrichtung haben. Es ist nicht ausgeschlossen, dass die orthopädietechnische Einrichtung hauptsächlich durch Biosignale gesteuert wird.

**In** einer Ausführungsform wird in einer Absenkphase eines Seitwärtsschrittes und/oder eines Verschwenkens in der Frontalebene eine Plantarflexion des Fußes durchgeführt, vorzugsweise in Kombination mit einer Knie- und Hüftstreckung. **In** der Absenkphase ist die Schrittlänge im Wesentlichen bereits erreicht und der Fuß wird zum Boden geführt. Die Steuerung des Fußes kann dabei derart gestaltet sein, dass der Initialkontakt mit dem Vorfuß erfolgt. Dies kann zum Beispiel durch eine Plantarflexion erreicht werden. Die Plantarflexionsbewegung kann gestoppt werden, sobald der Bodenkontakt erreicht ist. Es ist möglich, dass die Plantarflexion in Abhängigkeit des Oberschenkelwinkels, des Hüftwinkels und/oder des Kniewinkels gesteuert wird. Alternativ oder ergänzend kann die Plantarflexion in Abhängigkeit der Seitwärtsneigung und/oder Bewegung gesteuert werden, insbesondere das Ausmaß. Die Plantarflexion kann auch aus einer Kraft-Verschiebungs-Charakteristik resultieren, wobei diese Charakteristik ebenfalls auf Basis anderer Messgrößen variiert werden kann, zum Beispiel ein Neutralpunkt, eine Position die der Fuß kraftfrei einnehmen würde, verändert wird. Vorzugsweise wird ein Neutralpunkt in der Absetzphase von einer neutralen Fußstellung hin zu einer plantar flektierten Stellung verschoben. Mittels einer Plantarflexionsbewegung in der Absetzphase ist es möglich, dass der Bodenkontakt frühzeitig hergestellt wird. Die Plantarflexion kann so gesteuert werden, dass der Körperschwerpunkt bei einem Seitwärtsschritt weniger oder gar nicht abgesenkt werden muss, um den Bodenkontakt nach einer Schwungphase herzustellen. Bei einer Seitwärtsneigung und neutraler Fußstellung verkürzt sich die funktionale vertikale Beinlänge gegenüber dem gestreckten vertikalen Stehen. Dieser Längenunterschied kann durch eine Plantarflexion teilweise oder vollständig ausgeglichen werden, insbesondere in einer Absetzphase. Während der Belastung des Fußes wird vorzugsweise eine Dorsalextension gegen einen Bewegungswiderstand zugelassen, insbesondere so, dass spätestens bei vollständiger Lastübernahme die Fußsohle vollständig auf dem Boden aufliegt. Der Bewegungswiderstand kann bei Initialkontakt gegenüber dem Stehen vergleichsweise gering sein. Der Bewegungswiderstand kann zudem mit der Belastung, aber auch mit abnehmender Verschwenkung in der Frontalebene erhöht werden. Wird der Fuß im Zuge eines Seitwärtsschrittes nach oben oder unten gesetzt, beispielsweise um eine Höhendifferenz zu überwinden, kann diese durch eine entsprechende Steuerung des Fußes ausgeglichen werden.

In einer Ausgestaltung werden während eines Verschwenkens in der Frontalebene Knie, Hüfte und oder Fuß zumindest teilweise über die Orientierung des Unterschenkels, Oberschenkels und/oder des Hüftwinkels in der Sagittalebene gesteuert, insbesondere in der Schwungphase, aber auch in einer Be- und/oder Entlastungsphase. Es kann sich dabei um eine funktionelle kinematische Kopplung der Freiheitsgrade handeln. Auch andere Parameter können in diese Kopplung einfließen, zum Beispiel die Seitwärtsneigung und/oder Bewegung sowie deren zeitlicher Ablauf. Durch eine koordinierte Steuerung ist es möglich, den Fuß präzise zu positionieren und beispielsweise über die Oberschenkelbewegung zu steuern. In einer möglichen Ausgestaltung ist die funktionelle Kopplung derart ausgeführt, dass der Fuß unter der Hüfte bleibt. Alternativ kann der Fuß in seiner anterior-posterioren Position zur Hüfte belassen werden. Befindet sich der Fuß beim Abheben zum Beispiel vor dem Körper, kann er auch während der Schwungphase eines Seitwärtsschrittes vor dem Körper gehalten werden. Die funktionelle Kopplung kann von der Verschwenkung in der Frontalebene abhängig sein.

Als Bewegungswiderstand ist insbesondere eine Reaktionskraft zu verstehen, die notwendig ist, um eine gewisse Lage zu halten und/oder eine Bewegung auszuführen. Gleiches gilt für ein Reaktionsmoment bei einer Verschwenkung. Die Reaktionskraft, bzw. das Reaktionsmoment, können somit neben einem möglichen konstanten Anteil unter anderem von der Position und/oder deren Ableitungen abhängig sein. Bei einer Sperre wird keine Bewegung zugelassen, womit die Reaktionskraft der aufgebrachten Kraft entspricht. Auch dabei handelt es sich um einen Bewegungswiderstand. Ein Bewegungswiderstand kann zum Beispiel eine geschwindigkeitsproportionale Dämpfung, eine lineare oder nichtlineare Elastizität, eine konstante Kraft oder eine Superposition dieser Verhalten sein, um nur einige zu nennen. Ein Bewegungswiderstand kann durch einen Mechanismus mit entsprechenden intrinsischen Eigenschaften realisiert werden, aber auch durch Steuerung oder Regelung eines passiven oder aktiven Aktuators. Ebenso ist eine Kombination von mehreren passiven und/oder aktiven Aktuatoren möglich. Mehrere Komponenten können seriell und/oder parallel zueinander angeordnet sein.

Ein aktiver Aktuator ist in der Lage Arbeit an seiner Umgebung zu verrichten und wird in der Regel mit elektrischer oder chemischer Energie gespeist. Es ist auch möglich, dass ein Aktuator potentielle Energie aufnimmt und zu einem späteren Zeitpunkt abgibt. Aktuatoren können über elektromechanische Antriebe, wie beispielsweise Motoren oder piezoelektrische Elemente, realisiert sein. Aktuatoren können als thermische Aktuatoren ausgebildet sein oder als Elemente, die sich unter Einfluss eines elektromagnetischen Felds oder eines Wärmestroms zusammenziehen, ausdehnen und/oder in ihrer Form verändern. Durch Mechanismen können in der orthopädietechnischen Einrichtung rotatorische Antriebe in lineare Bewegungen übersetzt sein und umgekehrt. Aktuatoren können hydraulische oder pneumatische Komponenten umfassen. Ebenso können Aktuatoren durch chemische Prozesse angetrieben sein, wie beispielsweise Verbrennungsmotoren oder Muskelfasern, bei denen durch eine chemische Bindung eine Längenänderung hervorgerufen wird. Bewegungswiederstände können unter anderem als Reibungsbremsen, Klemmmechanismen, hydraulische und/oder pneumatische Dämpfer mit newtonschen und/oder nicht newtonschen Fluiden, magnetorheologische Dämpfer oder Bremsen, Magnetpulverbremsen, Hysterese- und/oder Wirbelstrombremsen, lineare und nichtlineare Feder, träge Massen oder andere Bremsmechanismen ausgebildet sein. Über Anschläge, Verriegelungs- und/oder Entriegelungsmechanismen, insbesondere mit Formschluss, kann der Bewegungswiderstand im Sinne einer Sperre und/oder eines Entriegelns verändert werden. Die orthopädietechnische Einrichtung kann auch über zumindest einen Stimulator verfügen. Insbesondere kann der zumindest eine Aktuator als Stimulator ausgebildet sein und einen Bewegungswiderstand verändern und/oder eine Bewegung steuern, zum Beispiel durch die elektrische Stimulation eines Muskels, die zu dessen Anspannung führt. Ein Muskel kann direkt stimuliert werden. Alternativ oder ergänzend können den Muskel innervierende Nerven und/oder Teile des zentralen und/oder peripheren Nervensystems stimuliert werden. Eine Stimulation kann invasiv und/oder nicht invasiv erfolgen. Eine Stimulation kann vor allem durch elektrische Signale, unter anderem aber auch mechanisch, thermisch und/oder chemisch erfolgen. Ein Beispiel ist die funktionelle Elektrostimulation von Muskeln.

Ein Aktuator kann so geregelt werden, dass eine Position, ein Winkel und/oder eine Zielgeschwindigkeit vorgegeben wird. Auch zeitliche Verläufe der Zielgrößen können vorgegeben werden. Es ist auch möglich einen Aktuator so zu regeln, dass ein Moment und/oder eine Kraft und/oder ein entsprechender Verlauf vorgegeben werden. Alternativ oder ergänzend kann ein Aktuator so gesteuert werden, dass er einem Kraft-Verschiebungs-Gesetzt folgt, das heißt, dass in Abhängigkeit einer Verschiebung und/oder Verdrehung sowie deren zeitlichen Änderungen eine Kraft und/oder ein Moment erzeugt wird und vice versa. Mittels solch einer Steuerung kann beispielsweise das Verhalten einer Feder oder eines Dämpfers nachgebildet werden.

In einer Ausführung werden bei einer Seitwärtsbewegung und/oder Seitwärtsverschwenkung zumindest ein Hüftwinkel, ein Oberschenkelwinkel, ein Kniewinkel und/oder ein Unterschenkelwinkel in der Sagittalebene und/oder deren zeitlicher Verlauf für die Steuerung herangezogen.

Eine Verschwenkung kann eine Rotation, eine Translation oder eine Kombination aus Rotation und Translation sein. Eine Verschwenkung kann zwischen zwei oder mehreren Komponenten ermittelt werden oder in Relation zu einem Bezugssystem, zum Beispiel einem Inertialsystem. Eine Verschwenkung kann sowohl eine konkrete Lage, eine Änderung der Lage über der Zeit oder aber auch ein zeitlicher Verlauf von Lagen und/oder Lageänderungen sein. Eine Seitwärtsbewegung kann ebenfalls eine Rotation, Translation oder eine Kombination aus Rotation und Translation sein.

Die Steuerung von Widerständen und/oder Bewegungen kann auch aufgrund von komplexen Kriterien oder Berechnungsvorschriften erfolgen, bspw. mittels Verfahren der künstlichen Intelligenz. Die Anpassung von Steuergrößen kann kontinuierlich, zum Beispiel als stetige Funktion von Sensorgrößen, in diskreten Schritten und/oder zu diskreten Zeitenpunkten, respektive Ereignissen erfolgen. Algorithmen zur Steuerung sowie Kriterien für die Steuerung können selbstlernend und/oder autoadaptiv sein.

Es kann sowohl eine Verschwenkung in der Frontaleben gesteuert werden als auch Verschwenkungen in anderen Richtungen und Ebenen auf Basis unter anderem einer Verschwenkung in der Frontalebene.

Die für die orthopädietechnische Einrichtung beschriebenen Steuerungen bei Verschwenkung in Medialrichtung können auch bei Verschwenkung in Lateralrichtung angewendet werden und vice versa. Es ist auch möglich bei Verschwenkung in Medialrichtung die orthopädietechnische Einrichtung anders anzusteuern als bei Verschwenkung in Lateralrichtung und vice versa. Die Steuerung kann sich kontinuierlich mit dem Ausmaß der Verschwenkung ändern, insbesondere um bei Bewegungsabläufen, die nur teilweise in der Frontalebene stattfinden, die Steuerung kontinuierlich gegenüber der Steuerung einer Bewegung, die nicht in der Frontalebene stattfindet, anzupassen, insbesondere um Bewegungswiderstände und/oder Bewegungen zu steuern. In einer Ausführung wird die Steuerung eines Kniegelenks in der Schwungphase ausgehend von einer Steuerung für das Geradeausgehen mit zunehmender Verschwenkung in der Frontalebene, zum Beispiel der Verschwenkung in der term inalen Standphase, verändert und an eine Schwungphasen-Steuerung für das Seitwärtsgehen angepasst. Eine sukzessive Anpassung kann auch während des Abrollens in der Standphase erfolgen. Alternativ kann eine Anpassung in mehreren diskreten Schritten erfolgen.

In einer Ausführung wird ein Verschwenken in der Frontalebene erkannt und ein oder mehrere damit verbundene Parameter für die Auswertung zu einem späteren Zeitpunkt und/oder die Übertragung an eine andere Komponente gespeichert. Zum Beispiel werden die Anzahl der Seitwärtsschritte und/oder ihre Schrittlänge detektiert, an einen Activity-Tracker übermittelt und dort angezeigt.

In einer Ausführung umfasst die Steuerung zumindest einen autoadaptiven oder selbstlernenden Algorithmus, der zumindest einen Parameter der Steuerung anpasst, wobei die Anpassung aufgrund einer erfassten Verschwenkung in der Frontalebene erfolgt und/oder der zumindest eine Parameter die Steuerung bei einer Verschwenkung in der Frontalebene beeinflusst.

Die orthopädietechnische Einrichtung der unteren Extremität mit einem Oberteil und einem Unterteil, die gelenkig um zumindest eine Schwenkachse miteinander unter Ausbildung eines Gelenkes aneinander gelagert sind und zumindest einem Aktuator, der mit einer Steuerungseinrichtung gekoppelt ist, die auf der Grundlage von Sensordaten zumindest eines mit der Steuerungseinrichtung gekoppelten Sensors den Aktuator aktiviert oder deaktiviert, um einen Verschwenkungswiderstand oder eine Relativbewegung des Oberteils zu dem Unterteil zu beeinflussen, sieht vor, dass der zumindest eine Sensor ausgebildet und eingerichtet ist, Sensordaten über eine Orientierung und/oder Verlagerung der orthopädietechnischen Einrichtung in der Frontalebene zu erfassen und dass die Steuerungseinrichtung eingerichtet ist, auf der Grundlage der Orientierung und/oder Verlagerung in der Frontalebene den Aktuator zu aktivieren, zu deaktivieren oder einen Sollwert für den Aktuator zu modulieren. Über den Aktuator wird das Oberteil relativ zu dem Unterteil bewegt, eine Bewegung gesperrt oder eine Bewegung zwischen dem Oberteil und dem Unterteil moderiert oder moduliert. Dies erfolgt durch das Einbringen von Energie in das System. Wird beispielsweise ein künstliches Kniegelenk als orthopädietechnische Einrichtung eingebeugt, verkürzt sich die funktionale Beinlänge. Umgekehrt wird bei einer Extension oder Streckung des künstlichen Kniegelenks die funktionale Beinlänge vergrößert. Bei der Erkennung einer Seitwärtsneigung innerhalb der Frontalebene kann sowohl das Standbein als auch das Schwungbein Gegenstand der Neigungserkennung sein. Ist das nicht versorgte Bein das Standbein, führt eine Neigung in der Frontalebene zu Beginn eines Seitwärtsschrittes zu einer Verkürzung der Gesamtbeinlänge, beispielsweise durch eine aktive Einbeugung eines künstlichen Kniegelenkes oder eine Verringerung des Flexionswiderstandes. Wird das versorgte Bein dann wieder abgesetzt, wird die Veränderung der Lage in der Frontalebene detektiert und die Beinlänge vergrößert und/oder der Flexionswiderstand erhöht bei Verringerung eines Extensionswiderstandes.

Der zumindest eine Sensor ist in einer Ausgestaltung als **IMU** ausgebildet und an dem Oberteil oder dem Unterteil befestigt. Insbesondere ist die orthopädietechnische Einrichtung nur mit einem **IMU** oder ausschließlich mit **IMU** als Sensoren zur Erfassung von Informationen hinsichtlich der Orientierung in der Frontalebene und in anderen Raumebenen ausgestattet. Über die **IMU** ist es möglich Lagen, Orientierungen und Beschleunigungen des Oberteils und/oder des Unterteils zu erfassen oder zu berechnen. Bei der Verwendung von zwei **IMU,** von denen eine dem Unterteil und die andere dem Oberteil zugeordnet ist, kann der Winkel zwischen dem Oberteil und dem Unterteil aus den beiden Absolutwinkeln oder Raumlagewinkeln in den jeweiligen Ebenen errechnet werden. Grundsätzlich ist auch eine Kombination mit anderen Sensoren möglich, die an der orthopädietechnischen Einrichtung und/oder dem Nutzer der orthopädietechnischen Einrichtung angeordnet sind und entsprechende Sensordaten ermitteln. Aus den Sensordaten können unabhängig von dem Aufbau der Sensoren Kriterien oder errechnete Größen ermittelt werden, zum Beispiel eine Projektion in einer Ebene, ein Krafteinleitungspunkt oder dergleichen, die zu einer Aktivierung oder Deaktivierung des Aktuators oder zu einer Veränderung eines Sollwertes für den Aktuator genutzt werden.

In einer Weiterbildung ist zumindest ein Kraftsensor, Beschleunigungssensor, Winkelsensor und/oder Momentensensor an dem Oberteil und/oder dem Unterteil angeordnet. Ein Kraftsensor kann beispielsweise zu Erfassung eines Bodenkontaktes ausgebildet sein. Ein kompressibles Element, ein verformbares oder verlagerbares Element oder auch ein elastisch gelagertes Element kann auf einen Kraftsensor oder einen als Kraftsensor dienenden Kontaktschalter einwirken, um beispielsweise zu erfassen, ob sich das jeweilige Bein in einer Standphase oder in einer Schwungphase befindet.

Der Aktuator kann auch auf ein Verriegelungselement einwirken, das eine mechanische, formschlüssigen Verriegelung oder Sperre des Oberteils relativ zu dem Unterteil bewirkt, sodass ein maximaler Widerstand gegen eine Verschwenkung bereitgestellt wird. Die formschlüssige Verriegelung kann richtungsabhängig sein, beispielsweise wie bei einer Umschaltknarre, sodass beispielsweise immer eine Extension möglich ist, auch wenn eine Fiktion gesperrt ist, oder umgekehrt.

Nachfolgend werden Ausführungsbeispiele der Erfindung anhand der Figuren näher erläutert. Es zeigen:
Es zeigen:
Figur 1 - eine schematische Darstellung eines Prothesenbeines;
Figur 2 - eine schematische Darstellung einer KAFO;
Figur 3 - einen ersten Bewegungsablauf;
Figur 4 - einen zweiten Bewegungsablauf;
Figur 5 - einen Bewegungsablauf für das Gehen um die Kurve
Figur 6 - das Anheben eines versorgten Beines;
Figur 7 - einen Seitwärtsschritt mit einem unversorgten Bein;
Figur 8 - einen Seitwärtsschritt mit einem versorgten Bein;
Figur 9 - eine Fußbewegung bei einem Seitwärtsschritt;
Figur 10 - das seitliche Übersteigen eines Hindernisses; sowie
Figur 11 - unterschiedliche Bewegungsabläufe.

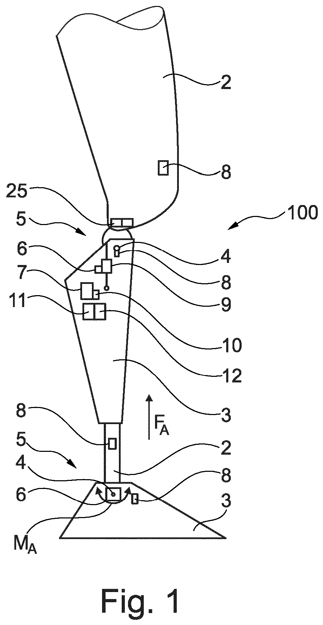

Figur 1 zeigt eine schematische Darstellung einer orthopädietechnischen Einrichtung 100 in Gestalt eines Prothesenbeines mit einem ersten Oberteil 2 in Gestalt eines Oberschenkelschaftes und einem ersten Unterteil 3 in Gestalt eines Unterteils eines Prothesenkniegelenkes 5. Das Oberteil 2 ist schwenkbar zu dem Prothesenunterteil 3 um eine Schwenkachse 4 gelagert. An dem Oberteil 2 sind Befestigungseinrichtungen 25 zur Festlegung des Oberschenkelschaftes an dem Prothesenkniegelenk 5 angeordnet oder ausgebildet. Die Befestigungseinrichtungen 25 sind beispielsweise ein Pyramidenadapter mit einer entsprechenden Aufnahme. Das erste Unterteil 3 in Gestalt eines Unterschenkelteils weist ein Unterschenkelrohr an seinem distalen Ende auf, das wiederum als zweites Oberteil 2 für eine gelenkige Verbindung mit einem Prothesenfuß als zweites Unterteil 3 dient. Der Prothesenfuß 3 ist schwenkbar um die Knöchelgelenksachse als zweite Schwenkachse 4 gelagert. Die schwenkbare Verbindung von Unterschenkelrohr und Prothesenfuß bildet das Knöchelgelenk 5 aus. Somit weist die orthopädietechnische Einrichtung 100 zwei Oberteile 2 und zwei Unterteile 3 auf, wobei das Unterschenkelteil als Verbindung zwischen den beiden Schwenkachsen 4 einteilig oder mehrteilig ausgebildet sein kann und je nach Betrachtungsweise einmal das Unterteil und einmal das Oberteil ist.

Die gelenkige Verbindung von Oberteil 2 und Unterteil 3 um die jeweilige Schwenkachse 4 bildet das jeweilige Gelenk 5 aus. **In** dem dargestellten Ausführungsbeispiel ist zwischen dem Oberteil 2 und dem Unterteil 3 des Kniegelenkes eine Widerstandseinrichtung 9 in Gestalt eines verstellbaren Dämpfers angeordnet. Die Widerstandseinrichtung 9 stützt sich mit einer proximalen Anschlusseinrichtung an dem Oberteil 2 und mit einer distalen Anschlusseinrichtung an dem Unterteil 3 ab. Die Widerstandseinrichtung 9 ist in dem Ausführungsbeispiel als passives Bauteil ausgebildet und beeinflusst eine Verschwenkbewegung des Oberteils 2 relativ zu dem Unterteil 3 um die Schwenkachse 4 sowohl in Flexionsrichtung als auch in Extensionsrichtung durch Umwandlung kinetischer Energie in Wärmeenergie. Der Widerstandseinrichtung 9 ist ein Aktuator 6 zur Verstellung des jeweiligen Widerstandes zugeordnet. Der Aktuator 6 wirkt auf die Widerstandseinrichtung 9 entsprechend dem Wirkprinzip ein. Ist die Widerstandseinrichtung 9 beispielsweise als eine pneumatische oder hydraulische Dämpfereinrichtung ausgebildet, verändert der Aktuator 6 den Strömungsquerschnitt der Leitung von einer Extensionskammer in die Flexionskammer und zurück, um so den jeweiligen Strömungsquerschnitt eines Überströmkanals zu vergrößern und zu verkleinern. Dadurch wird der Strömungswiderstand verkleinert oder vergrößert. Alternativ oder ergänzend zu einer Veränderung des Strömungsquerschnittes kann der Aktuator 6 als verstellbarer Magnet ausgebildet sein, z.B. als Elektromagnet, der auf eine magnetorheologische Flüssigkeit einwirkt. Durch Veränderung des Magnetfeldes verändert sich die Viskosität der magnetorheologischen Flüssigkeit, sodass über die Veränderung der Viskosität der Verschwenkungswiderstand verändert wird. Die Widerstandseinrichtung 9 kann auch als ein Elektromotor ausgebildet sein, der im Generatorbetrieb betrieben werden kann, bei der der Flexionswiderstand und/oder der Extensionswiderstand durch eine entsprechende Generatorregelung verändert wird. **In** diesem Fall ist in der Regel der Generator der Aktuator. Ist eine rein mechanische Bremse, beispielsweise ein Reibungsbremse, vorgesehen, bei der Bremsbeläge gegen eine sich bewegende Komponente gepresst werden, ist der Aktuator der Motor oder Antrieb, mit dem die Bremsbeläge gegen die Komponente gedrückt werden.

Alternativ oder ergänzend zu einer rein passiven Ausgestaltung der Widerstandseinrichtung kann der Aktuator 6 auch als aktives Element, z.B. als Elektromotor ausgebildet sein, um eine Relativbewegung des Oberteils 2 zu dem Unterteil 3 nicht nur zu beeinflussen, sondern auch aktiv hervorzurufen. Alternativ zu einer Ausgestaltung als Elektromotor kann der Aktuator 6 auch andere Antriebseinrichtungen oder -prinzipien zur Freisetzung gespeicherter Energie nutzen.

Der Aktuator 6 wird über eine Steuerungseinrichtung 7 aktiviert, deaktiviert oder moduliert. Je nach Signal von der Steuerungseinrichtung wird die Flexion und/oder Extension beeinflusst und gegebenenfalls blockiert. Durch die Steuerungseinrichtung 7 mit dem entsprechenden Signal wird das Bewegungsverhalten des jeweiligen Gelenkes 5 während des Gehens, Stehens oder einer anderen Nutzung eingestellt. Der Steuerungseinrichtung 7 sind Sensoren 8 zugeordnet, die auf der gesamten orthopädietechnischen Einrichtung 100 angeordnet sind. Die Sensoren 8 liefern entsprechende Daten drahtlos oder über Kabelverbindungen an die Steuerungseinrichtung 7. Die Daten der Sensoren 8 können vorverarbeitet und/oder in der Steuerungseinrichtung 7 selbst verarbeitet werden. In der Steuerungseinrichtung 7 sind Prozessoren, Speicher und alle anderen notwendigen Komponenten vorhanden oder mit ihr gekoppelt, um die Sensordaten auszuwerten und auf der Basis dieser Auswertung eine entsprechende Aktivierung, Deaktivierung oder Modellierung des Aktuators und damit der Widerstandseinrichtung 9 vorzunehmen.

Die Steuerungseinrichtung 7 weist insbesondere auch eine Speichereinrichtung 10 auf und kann mit einem Sender 11 und einem Empfänger 12 gekoppelt sein, um Sensordaten, Programme, Zugriffsrechte, Einstellungen, Änderungen von Einstellungen, Updates oder anderes auf externe Komponenten oder auf Komponenten innerhalb der orthopädietechnischen Einrichtung zu übertragen. Die Sensoren 8 erfassen während der Nutzung der orthopädietechnischen Einrichtung sämtliche relevanten Parameter, beispielsweise Kräfte, Momente, Beschleunigungen, Temperaturen, Zeiten, Orientierungen im Raum, Verformungen, Bewegungszeiträume, Nutzungszeiträume, Distanzen, relative Bewegungen, Interaktionen mit der Umgebung, Spannungen, Ströme, Biosignale, elektromagnetische Strahlungen und dergleichen. Insbesondere sind die Sensoren 8 oder Sensoreinrichtungen als Komponenten ausgebildet, die eine Winkelstellung der Komponenten zueinander und/oder eine Raumlage oder eine Orientierung im Raum erkennen. Zusätzlich sind die Sensoren 8 zur Erfassung von Axialkräften FA und Momenten MA ausgebildet. Die Kräfte und Momente werden überall dort ermittelt, wo die Erfassung sinnvoll und notwendig ist, auch wenn diese Kräfte und Momente nur im Zusammenhang mit dem Knöchelgelenk eingezeichnet sind. Nicht alle Sensoren 8 können alle Parameter erfassen, die Anordnung und Ausgestaltung der Sensoren richtet sich nach den jeweils zu erfassenden Parametern.

Aus Sensorwerten können auch abgeleitete Größen errechnet werden. Beispielsweise können aus Kraft- und/oder Momentenkomponenten Hebelarme zu gewissen Punkten und/oder Kraftangriffspunkten berechnet werden, Sensorwerte zu Kenngrößen fusioniert werden, wie beispielsweise in IMUs (Inertial Measurement Units), aus Verformungen auf Kräfte rückgerechnet werden und/oder aus mehreren Abständen durch Triangulation auf eine Position zurück gerechnet werden. Derartige errechnete Größen sind in den beschriebenen Ausführungen mit eingeschlossen und können für die Steuerung der orthopädietechnischen Einrichtung herangezogen werden, insbesondere zur Steuerung von Bewegungsabläufen mit einer Verschwenkung in der Frontalebene.

In dem Ausführungsbeispiel ist an dem Knöchelgelenk als Aktuator 6 ein Elektromotor angeordnet, über den je nach Bedarf über den Generatorbetrieb eine Widerstandseinrichtung und im Motorbetrieb eine Unterstützung oder eine aktive Verlagerung des Prothesenfußes relativ zu dem Unterschenkelteil um die Schwenkachse 4 bereitgestellt wird.

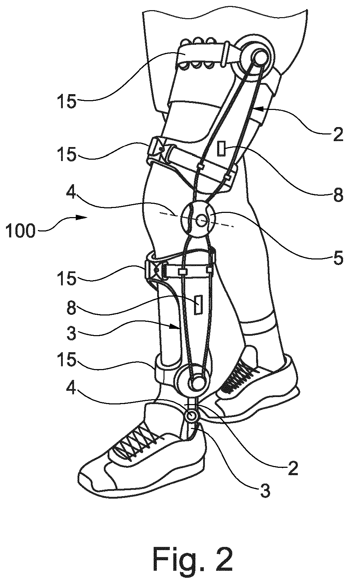

In der Figur 2 ist eine orthopädietechnische Einrichtung 100 als eine Orthese der unteren Extremität in einem angelegten Zustand gezeigt. Es handelt sich hierbei um eine KAFO (Knee Ankle Foot Orthosis), bei der ein erstes Oberteil 2 in Gestalt einer Oberschenkelschiene über Befestigungseinrichtungen 15 in Gestalt von Gurten an einem Oberschenkel festgelegt ist. Ein erstes Unterteil 3 in Gestalt einer Unterschenkelschiene ist ebenfalls über Befestigungseinrichtungen 15 an einem Unterschenkel eines Nutzers angeordnet. Die Oberschenkelschiene und die Unterschenkelschiene sind um eine Schwenkachse 4 unter Ausbildung eines Orthesenkniegelenkes 5 schwenkbar aneinander befestigt. An oder in dem Orthesenkniegelenk 5 sind die in der Figur 1 erläuterten Komponenten und technischen Einrichtungen wie Aktuator, Widerstandseinrichtung, Steuerungseinrichtung, Schnittstellen und dergleichen angeordnet. Die Sensoren 8 sind schematisch dargestellt. Die zweite Schwenkachse 4 im Bereich des natürlichen Knöchelgelenkes verbindet die Unterschenkelschiene als zweites Oberteil 2 mit einem Fußteil als zweites Unterteil 3. Die Einrichtung zur Beeinflussung des Prothesenknöchelgelenkes hinsichtlich des Widerstandes in Richtung der Plantarflexion oder der Dorsalflexion ist im Bereich des Orthesenknöchelgelenkes untergebracht. Auch hier können passive Widerstandseinrichtungen und/oder aktive Antriebe oder Aktuatoren vorgesehen sein.

Sowohl bei der Ausgestaltung als Prothese als auch bei der Ausgestaltung als Orthese können bei mehreren Gelenken 5 und entsprechenden Widerstandseinrichtungen die Aktuatoren 6 zur Beeinflussung der Verschwenkbewegung um die jeweilige Schwenkachse 4 von einer gemeinsamen Steuerungseinrichtung 7 gesteuert werden. Ebenso ist es möglich, dass mehrere Steuerungseinrichtungen 7 ausgebildet oder angeordnet sind, um die orthopädietechnische Einrichtung 100 entsprechend zu steuern.

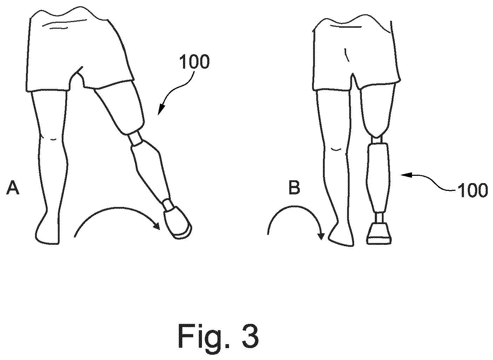

In der Figur 3 ist schematisch ein erster Bewegungsablauf einer Person mit einer orthopädietechnischen Einrichtung 100 in Gestalt eines Prothesenbeins, ähnlich dem in der Figur 1, dargestellt. Die nutzende Person der orthopädietechnischen Einrichtung 100 steht im Wesentlichen gerade und aufrecht. In der dargestellten Stellung ist das vom Nutzer aus gesehen linke Bein das versorgte Bein. Eine Vorwärtsbewegung wäre eine Bewegung aus der Blattebene heraus. Möchte nun die nutzende Person eine seitliche Bewegung von sich aus nach links durchführen, wird zunächst das linke, versorgte Bein mit der orthopädietechnischen Einrichtung 100 angehoben und lateral nach außen bewegt und/oder eine Abduktionsbewegung durchgeführt, was in der linken Darstellung in der Situation dargestellt ist. Nach dem Aufsetzen des Prothesenfußes der orthopädietechnischen Einrichtung 100 verlagert die nutzende Person das Gewicht auf die versorgte Seite und zieht ihren rechten, unversorgten Fuß neben den Prothesenfuß. Die Bewegung findet überwiegend in der Frontalebene statt, was der Blattebene entspricht. Nach dem Anheben des Prothesenfußes wird z.B. eine Flexionsbewegung innerhalb des Knies erleichtert oder eingeleitet, indem der Flexionswiderstand verringert oder eine aktive Flexionsunterstützung durch den nicht dargestellten Aktuator initiiert wird. Die Erkennung dieser Bewegung erfolgt beispielsweise durch eine Überwachung des Axialkraftverlaufes innerhalb des Prothesenunterteils oder des Prothesenfußes in Verbindung mit der Überwachung einer Bewegung und/oder Stellung der orthopädietechnischen Einrichtung in der Frontalebene. Wird eine seitliche, abduzierende Bewegung ausgeführt und der Prothesenfuß lateral nach außen verlagert, auch ohne dass eine Neigung oder Bewegung innerhalb der Sagittalebene oder nach vorne stattfindet, wird ein Flexionswiderstand verringert. Alternativ oder ergänzend wird darüber hinaus eine Extensionsbewegung innerhalb des Prothesenkniegelenkes verzögert oder unterbunden, sodass ein Schritt zur Seite möglich ist. Sobald eine Axialbelastung oder ein Aufsetzen des Fußes erfolgt, wird in einer Ausführungsform der Flexionswiderstand entweder erhöht oder eine Extension bewirkt.

Bei einer rein seitlichen Verschwenkung oder Neigung ist es möglich, eine Plantarflexion zu unterbinden und auch eine Dorsalflexion zu bewirken, damit der Prothesenfuß vollflächig oder mit einer geraden Sohle im Wesentlichen parallel zum Untergrund aufgesetzt werden kann. Alternativ kann die Bewegung von Stellung A zu Stellung B bei einem aktiven Fuß mit einer Plantarflexion verbunden sein, sodass nach dem Aufsetzen oder beim Aufsetzen zunächst eine Fußspitze aufsetzt und bei einer zunehmenden Belastung eine Dorsalflexion stattfindet. Es ist auch möglich dass der Fuß während der Bewegung von einer Stellung A zu Stellung B in einer leicht nach unten zeigender Position gehalten wird oder in diese gebracht wird.

Nach der Belastung, die in der Position B dargestellt ist, wird der Flexionswiderstand auf einem hohen Niveau gehalten und/oder eine Extension durchgeführt. Bei einer aktiven Prothese mit Antrieben wird zunächst eine Knieflexion eingeleitet. Der Kniewinkel kann in einer bestimmten Stellung während der Abduktion konstant gehalten werden, bis die Hüfte gestreckt wird. Es ist möglich, dass zumindest ein Freiheitsgrad mit zumindest einem Gelenkwinkel und/oder einem Segmentwinkel, welche von der nutzenden Person hinreichend angesteuert werden können, kinematisch gekoppelt sind. Eine kinematische Kopplung kann unter anderem eine holonome oder nichtholonome Zwangsbedingung sein. Alternativ oder ergänzend ist es möglich, dass mehrere Freiheitsgrade untereinander einer kinematischen Kopplung folgen. Alternativ oder ergänzend zu einer kinematischen Kopplung können mehrere Freiheitsgrade einer Kraft- und/oder Momentenkopplung unterliegen, wodurch eine harmonische Steuerung mehrerer Freiheitsgrade erzielt werden kann.

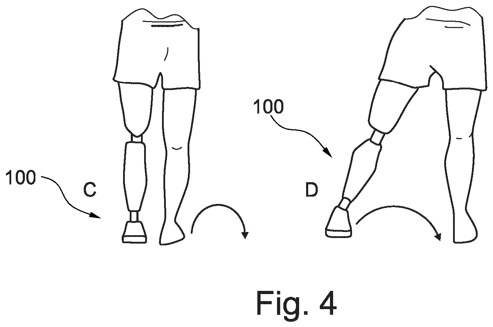

In der Figur 4 ist eine alternative Situation gezeigt, bei der zunächst die versorgte Seite mit der orthopädietechnischen Einrichtung 100 belastet bleibt und das gesunde, unversorgte Bein zur Seite verlagert wird. Nach dem Aufsetzen des gesunden Beines befindet sich die versorgte Seite in einem in der Frontalebene geneigten Zustand innerhalb der Standphase. Die Neigung innerhalb der Frontalebene wird detektiert, ggf. auch eine Neigung in der Sagittalebene. Ab einem bestimmten Neigungswinkel wird bei einer aktiven Prothese zunächst eine Knieflexion eingeleitet. Anschließend, in Abhängigkeit von einem Zeitfaktor oder der Adduktion oder des Heranziehens des versorgten Beines an die unversorgte Seite oder zur Körpermitte, findet eine Extension oder eine Streckung innerhalb des Prothesenkniegelenkes statt.

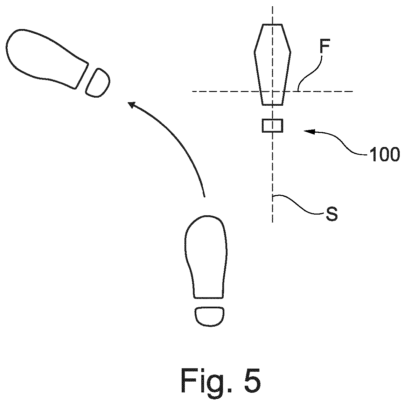

In der Figur 5 ist ein Bewegungsmuster gezeigt, bei dem ein Nutzer einer orthopädietechnischen Einrichtung 100 um eine Kurve gehen möchte, in dem dargestellten Ausführungsbeispiel eine Linkskurve. Dabei bleibt das versorgte Bein mit der orthopädietechnischen Einrichtung 100 aufgesetzt und befindet sich in der Standphase. Das unversorgte Bein wird bis an die Frontalebene F herangeführt und gleichzeitig nach links verdreht. Bei einer herkömmlichen Prothese oder Orthese aus dem Stand der Technik würde es in einer solchen Situation keine Freischaltung eines Gelenkes, insbesondere keine Reduzierung eines Flexionswiderstandes des Kniegelenkes bewirken, da nur eine geringe Vorwärtsrotation innerhalb der orthopädietechnischen Einrichtung 100 detektierbar ist, bzw. der Kraftangriffspunkt an dem Fuß in der Fußmitte bleibt. Eine Flexion innerhalb des Kniegelenkes würde nicht ausgelöst werden. Erfindungsgemäß wird jedoch eine Seitwärtsrotation und eine Neigung innerhalb der Frontalebene F der versorgten Seite erfasst. Bei dem seitlichen Schritt gemäß Figur 5, der angenähert der Situation in der Figur 4 entspricht, wird eine Neigung von dem Prothesenfuß aus gesehen in medialer Richtung detektiert, gegebenenfalls in Verbindung mit einer Vorwärtsrotation aus einer nach hinten geneigten Stellung des Unterschenkelteils und/oder des Prothesenschaftes. Bei einer Detektion einer geringen Vorwärtsrotation in Kombination mit einer Seitwärtsrotation und einer geneigten Stellung innerhalb der Frontalebene F wird der Flexionswiderstand verändert, insbesondere verringert, sodass das Bein mit der orthopädietechnischen Einrichtung leicht angehoben, flektiert und anschließend wieder aufgesetzt werden kann.

Zur Detektion einer Seitwärtsbewegung oder einer intendierten Seitwärtsbewegung ist es möglich, eine Kraftmessung innerhalb der distalen Endkomponente durchzuführen, beispielsweise in einer Fußplatte einer Orthese oder in einem Prothesenfuß. Der Krafteinleitungspunkt wandert bei einer Bewegung innerhalb der Frontalebene von innen nach außen bzw. von außen nach innen im Knöchel und nicht nach vorn und hinten, sodass aus dem Verlauf des Krafteinleitungspunktes innerhalb der orthopädietechnischen Einrichtung 100 Rückschluss darauf genommen werden kann, welche Bewegung gerade ausgeführt wird oder werden soll. Alternativ oder ergänzend kann die Orientierung und/oder Verkippung des Kraftvektors und damit dessen zeitliche Veränderung herangezogen werden, um eine Seitwärtsbewegung zu detektieren, insbesondere eine Verkippung in der Frontalebene. Es ist auch möglich, Momente in der Frontalebene zu messen und aufgrund einer Zu- oder Abnahme auf eine Verschwenkung zu schließen.

Bei der Steuerung der Widerstandseinrichtungen oder der Bewegungen in den Gelenken der orthopädietechnischen Einrichtung 100 der unteren Extremität werden zusätzlich zu den Bewegungsabläufen innerhalb der Sagittalebene diejenigen Bewegungsabläufe und Zustände oder Orientierungen erfasst, die in der Frontalebene stattfinden. Die notwendigen Sensorsignale, die Rückschlüsse auf eine Bewegung innerhalb der Frontalebene und die Art der Bewegung innerhalb der Frontalebene ergeben, werden über die Sensoren 8, insbesondere über eine IMU abgeleitet. Zusätzlich zu Neigungen und Verschwenkungen innerhalb der Frontalebene können translatorische Bewegungen in bestimmten Ebenen innerhalb der Frontalebene über eine Wegintegration von Beschleunigungen ermittelt werden. Beschleunigungen können über eine IMU gemessen werden. Sofern die Beschleunigungen nicht in einem inertialfesten Koordinatensystem bestimmt werden, sind für die Integration im Allgemeinen die zusätzliche Bestimmung der Raumlage und eine Transformation in ein inertialfestes Koordinatensystem notwendig.

In der Figur 5 ist als Ausführungsbeispiel die Steuerung einer Beinprothese oder einer Orthese bei einer Richtungsänderung mit einer Außenrotation auf dem versorgten Standbein gezeigt. Die Rotation des versorgten Beines erfolgt dabei im Wesentlichen in der Hüfte. Der Prothesenfuß oder das Fußteil der Orthese rotiert in der Standphase nicht oder nur in einem zu vernachlässigenden Maße gegenüber dem Untergrund. Grundsätzlich wäre auch eine Innenrotation mit dem versorgten Bein möglich. Innenrotationen sind aber eher unüblich und führen insbesondere bei Patienten mit Prothesenbeinen zu unvorteilhaften Bewegungsmustern. Im Gegensatz zum Vorwärtsgehen rollt das versorgte Bein in der Standphase, insbesondere zum Ende der Standphase, nicht innerhalb der Sagittalebene nach vorne um das Knöchelgelenk, sondern innerhalb der Frontalebene zur Seite ab. Dabei ist es wünschenswert, dass das Kniegelenk am Ende der Standphase bei einer solchen Bewegung eine Einleitung der Schwungphase bei einem geringen Beugewiderstand ermöglicht oder dass bei einem aktiven Prothesenkniegelenk eine Flexionsbewegung eingeleitet oder zumindest unterstützt wird.

Daher ist es erfindungsgemäß vorgesehen, dass eine Schwungphaseneinleitung mit einer Verringerung des Flexionswiderstandes dann ermöglicht oder eingeleitet wird, wenn das Bein in der Frontalebene abduziert. Neben einer Seitwärtsbewegung, also einer Verschwenkung in dem Ausführungsbeispiel gemäß der Figuren 4 und 5, kann auch die Seitwärtsneigung als solche ausschlaggebend sein. Dabei wird die Neigung im Sinne einer Verkippung gegenüber der Vertikalen herangezogen, die auch unabhängig von der Richtung der Verkippung sein kann. Ergänzend kann gefordert sein, dass sich das Prothesenkniegelenk nicht hinter der Schwenkachse des Knöchelgelenkes befindet, also das Bein nicht nach hinten gekippt ist oder keine Rückwärtsbewegung stattfindet. Die Veränderung der Neigung soll insbesondere nicht innerhalb der posterioren Hemisphäre erfolgen, sondern nach Möglichkeit in der anterioren Hemisphäre oder bei einer Bewegung in Vorwärtsrichtung.

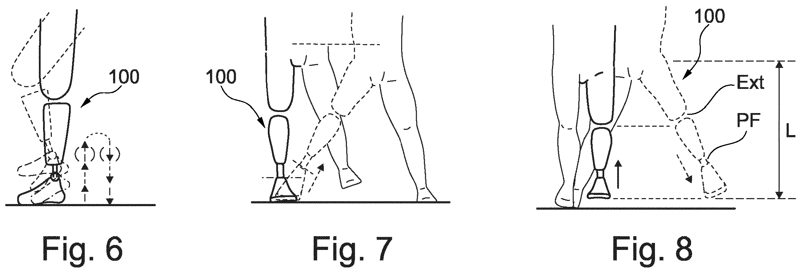

In der Figur 6 ist der Ablauf einer Anhebebewegung eines versorgten Beines, in dem dargestellten Ausführungsbeispiel eines Prothesenbeines, dargestellt. Grundsätzlich gelten die Ausführungen für ein Prothesenbein auch für eine orthetische Versorgung. Aus einer gestreckten Stellung, in der das Prothesenbein 100 auf dem Boden steht, wird eine Hüftbeugung durchgeführt, wodurch das Prothesenkniegelenk eingebeugt und der Prothesenfuß angehoben wird. Dabei wird das Knöchelgelenk im Wesentlichen senkrecht nach oben angehoben. Eine Plantarflexion findet in der ersten Anhebephase statt, sodass die Fußspitze nach unten fällt. Am Ende der Anhebebewegung ist die Fußspitze frei in der Luft in einer plantar flektierten Stellung und wird anschließend von einem Aktuator angehoben, sodass eine Dorsalflexion stattfindet. Umgekehrt wird zum Absetzen, beispielsweise bei oder nach einer Seitwärtsbewegung zunächst die Fußspitze abgesenkt oder eine Plantarflexion eingeleitet oder ermöglicht, beispielsweise durch Verringerung des Flexionswiderstandes. Nach dem Aufsetzen der Fußspitze an der gewünschten Position wird eine Dorsalflexion durch eine Belastung des versorgten Beines durchgeführt. In Abhängigkeit von der jeweils festgestellten Neigung in der Frontalebene und gegebenenfalls in Verbindung mit weiteren Sensorwerten werden entsprechende Aktuatoren aktiviert, deaktiviert oder Sollwerte für den Aktuator moduliert, um das Aufsetzen einfach zu gestalten und sicher zu ermöglichen.

In der Figur 7 ist eine Seitwärtsbewegung ähnlich der Figur 4 gezeigt. Zunächst wird das unversorgte Bein angehoben und in der Frontalebene seitlich von dem Prothesenfuß abgesetzt. Um den Körperschwerpunkt möglichst auf einem Niveau zu belassen, was durch die gestrichelte Linie dargestellt ist, wird im Verlauf der Seitwärtsbewegung bei einem Verschwenken des versorgten Beines in der Frontalebene eine Plantarflexion eingeleitet, beispielsweise durch einen Antrieb oder Aktuator, was zu einer Verlängerung der effektiven Beinlänge führt. Die Verlängerung ist notwendig, damit eine Verschwenkbewegung um den Aufsetzpunkt des Prothesenfußes herum stattfinden kann. Die Verlängerung der effektiven Beinlänge durch die Plantarflexion ist durch den verlängert dargestellten Prothesenfuß angedeutet. Wird die versorgte Seite dann wieder an den anderen Fuß herangezogen, muss dazu der Prothesenfuß angehoben werden. Die Entlastung des Prothesenfußes kann beispielsweise über einen Kontaktschalter oder einen Axialkraftsensor detektiert werden. Zur Erleichterung der Adduktionsbewegung findet eine Dorsalflexion statt, die ab einem bestimmten Neigungswinkel des versorgten Beines innerhalb der Frontalebene umgekehrt werden kann, was zu einer Plantarflexion führt, um ein sanftes Aufsetzen des Prothesenfußes zu ermöglichen.

In der Figur 8 ist die umgekehrte Bewegung dargestellt, nämlich das Versetzen der versorgten Seite von der unversorgten Seite weg. Dazu wird zunächst die versorgte Seite angehoben und innerhalb der Frontalebene von dem stehenden Fuß wegbewegt. Nach dem Anheben befindet sich der Prothesenfuß noch in der Ausgangsposition ohne Plantarflexion, das Kniegelenk ist eingebeugt. Dabei stellt sich eine effektive Beinlänge L ein, die während der Seitwärtsbewegung vorteilhafterweise beibehalten wird. Dies geschieht durch eine Extension Ext in dem Kniegelenk sowie eine Plantarflexion PF in dem Knöchelgelenk, sodass die Fußsohle ebenso wie der Hüftdrehpunkt während der Seitwärtsbewegung im Wesentlichen auf jeweils einem Niveau bleiben, was durch die gestrichelten Linien angedeutet ist.

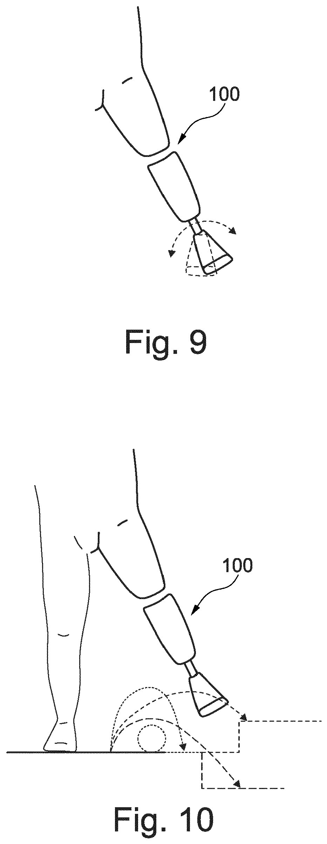

**In** der Figur 9 ist eine mögliche Verlagerung des Prothesenfußes bei einer seitlichen Abwinkelung des Prothesenbeines gezeigt. Während der Abspreizbewegung des Beines kann eine Supination oder eine Pronation des Prothesenfußes stattfinden, um eine Anpassung der Beinlänge ebenso wie eine Anpassung an die jeweilige Bewegungssituation und den Bewegungsverlauf vornehmen zu können. **In** der Figur 10 ist das Übersteigen eines Hindernisses dargestellt, wobei das Aufsetzen des Prothesenfußes auf der gleichen Ebene wie der des Standbeines erfolgen kann. Alternativ erfolgt das Absetzen auf einem höheren oder einem niedrigeren Niveau. Je nach Winkelstellung des versorgten Beines wird eine Aktivierung eines Aktuators durchgeführt, um die Stellung des Prothesenfußes an das jeweilige Aufsetzniveau anpassen zu können.

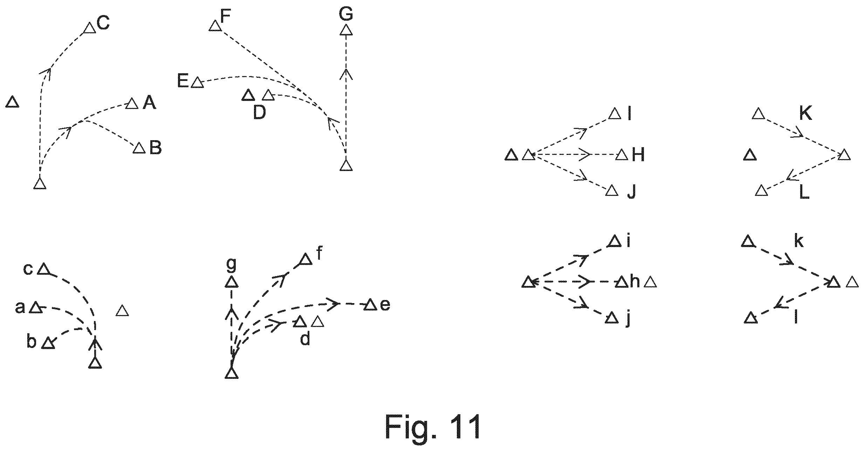

**In** der Figur 11 sind unterschiedliche Bewegungsmuster für eine seitliche Bewegung innerhalb der Frontalebene dargestellt. **In** der oberen Bewegungsreihe sind die Bewegungen von der versorgten Seite ausgeführt und mit Großbuchstaben gekennzeichnet, in der unteren Reihe ist die versorgte Seite das Standbein und die unversorgte Seite wird bewegt, was mit Kleinbuchstaben gekennzeichnet ist. Grundsätzlich sind die Bewegungen auch umgekehrt durchführbar. Bei der Bewegung A wird bei einem hinten stehenden Prothesenfuß, beispielsweise am Ende eines Schrittes, bei dem die unversorgte Seite vorne ist, die versorgte Seite sowohl nach vorne als auch seitlich bis auf die Höhe der unversorgten Seite bewegt, sodass sich eine Kurvenbewegung einstellt. Bei der Bewegung B findet eine Bewegungsumkehr der Vorwärtsbewegung statt, sodass die versorgte Seite, beispielsweise der Prothesenfuß, schräg hinter den unversorgten Fuß gestellt wird. Bei der Bewegung C wird die versorgte Seite geradlinig in der Sagittalebene bis zur Höhe des unversorgten Fußes geführt und dann schräg nach vorne abgesetzt. **In** allen drei Bewegungen findet eine Verschwenkung der versorgten Seite innerhalb der Frontalebene um einen Drehpunkt in dem Hüftgelenk statt.

Bei den Bewegungen D bis G ist die Ausgangsposition eine versorgte Seite mit einem Prothesenfuß schräg hinter der unversorgten Seite. Die versorgte Seite wird in der Bewegung D seitlich neben den unversorgten Fuß in einer Kreisbahnbewegung geführt, bei der Bewegung E vor den unversorgten Fuß im Rahmen eines Kreuzschrittes. Bei der Bewegung F wird der versorgte Fuß übergesetzt und schräg vor den unversorgten Fuß gesetzt. Bei der Bewegung G erfolgt eine geradlinige Bewegung aus einem breitbandigen Stand nach vorne. **In** allen Bewegungen befindet sich die versorgte Seite in der Ausgangsposition aufgrund des breitbeinigen Standes in einer in der Frontalebene geneigten Stellung, bei den Bewegungen E und F kehrt sich die Neigungsrichtung um, in der Position D ist am Ende der Bewegung die versorgte Seite senkrecht orientiert.

Die seitlichen Bewegungen von der unversorgten Seite weg sind in den Figuren I bis J gezeigt, wobei bei der Bewegung H die Bewegung vollständig in der Frontalebene stattfindet, bei den Bewegungen I und J erfolgt eine Bewegung schräg nach vorne bzw. schräg nach hinten. Bei den Bewegungen L und K werden vor bzw. hinter dem unversorgten Fuß stehender Prothesenfüße in die Frontalebene auf Höhe des unversorgten Fußes abgesetzt. Beide Füße befinden sich innerhalb der Frontalebene.

Die gleichen oder entsprechende Bewegungen werden in der unteren Reihe mit der unversorgten Seite ausgeführt, der Prothesenfuß oder der Fußteil der versorgten Seite ist dabei die stehende Komponente. Die Bewegungen sind entsprechend ausgeführt und können in beide Richtungen ausgeführt werden, also statt einem Heranziehen ein Wegsetzen und umgekehrt. Neigungen in der Frontalebene bei den Bewegungen mit der unversorgten Seite in der Schwungphase erfolgen über eine Drehung im Knöchelgelenk mit einer fixierten Position oder über eine Drehung um einen Fußaufsatzpunkt oder einem COP.

Die Einleitung der Schwungphase kann bei entsprechend starker Seitwärtsneigung innerhalb der Frontalebene oder bei einer entsprechenden Rotation oder Verschwenkung um einen distalen Drehpunkt mit weniger Vorwärtsneigung oder mit weniger Bewegung in die Vorwärtsrichtung stattfinden, wobei die jeweiligen Neigungen und auch die Verschwenkungsgeschwindigkeiten innerhalb der Sagittalebene und der Frontalebene unterschiedlich gewichtet sein können.

Zusätzlich zu Neigungen, Verschwenkungsgeschwindigkeiten und Neigungsgeschwindigkeiten sowie Richtungen können Kräfte und Momente auf die orthopädietechnischen Einrichtungen bei der Beeinflussung der Gelenkbewegungen berücksichtigt werden.

Wird nach der Einleitung einer Schwungphase wenn die versorgte Seite keinen Bodenkontakt mehr hat eine entsprechende Bewegung ausgeführt, kann diese Bewegung in Abhängigkeit von den erreichten Winkeln innerhalb der Frontalebene, gegebenenfalls in Verbindung mit den ermittelten Rotationswerten und Belastungswerten der Bewegungsumfänge und Verläufe innerhalb der Schwungphase verändert werden. Beispielsweise kann ein größerer, erreichbarer Flexionswinkel oder eine größere Flexion, erlaubt werden, wenn eine starke Neigung vorhanden ist und eine entsprechend enge Linkskurve oder Rechtskurve gegangen werden soll.

Neben der Beeinflussung der Flexion und Extension des Prothesenkniegelenkes oder Orthesenkniegelenkes der orthopädietechnischen Einrichtung 100 durch Aktivierung, Deaktivierung oder Modulierung des Aktuators kann ein Prothesenfuß oder ein Orthesengelenk ebenfalls entsprechend beeinflusst werden. Beispielsweise kann in der Standphase bei einer Detektion einer Neigung oder Verschwenkung innerhalb der Frontalebene eine Überrollcharakteristik ähnlich dem eines Gehens aktiviert werden, also eine Dorsalflexion zulassen oder eine Plantarflexion unterstützen, auch wenn keine Vorwärtsrotation stattfindet. Bei einem aktiven Prothesenfuß kann bei einem erkannten Richtungswechsel über die Seitwärtsneigung innerhalb der Frontalebene eine aktive Plantarflexion initiiert werden.

Bei einem wie oben beschriebenen Richtungswechsel ist es möglich, eine Flexion des Kniegelenkes und/oder eine Dorsalextension eines Fußes in der Standphase zu verlangsamen, zu beschränken oder dem aktiv entgegenzuwirken. Der Bewegungsimpuls kann dadurch besser in die neue Gehrichtung umgeleitet werden. Bei diesen sogenannten Bremsschritten, bei denen die prothetische Einrichtung durch das Körpergewicht belastet wird, wird in den ausschließlich die Sagittalbewegung berücksichtigenden Steuerungen ein uneingeschränktes Einbeugen des Kniegelenkes zugelassen. Bei einer erkannten Seitwärtsrotation, Verschwenkung und/oder Neigung wird hingegen der Flexionswiderstand erhöht, um ein weiteres Einbeugen zu verhindern. Bei einem aktiven Prothesenkniegelenk kann eine Extensionsbewegung eingeleitet werden. Ein Prothesenfuß oder ein Orthesenfuß wird hinsichtlich einer Dorsalflexion einen hohen Widerstand bereitstellen, sodass die Fußspitze nicht angehoben wird. Dafür werden die entsprechenden Widerstände einer Widerstandseinrichtung über den Aktuator eingestellt, gegebenenfalls wird eine aktive Plantarflexion bei aktiven Einrichtungen ausgeführt.

Bei rein passiven orthopädietechnischen Einrichtungen werden keine Bewegungen eingeleitet oder unterstützt, sondern nur unterschiedlich hohe Widerstände in den jeweiligen Bewegungsrichtungen um die jeweiligen Schwenkachsen bereitgestellt. Dadurch werden Verlagerungen zueinander begrenzt und der Bewegungsumfang oder Range of Motion eingeschränkt bzw. Bewegungsgeschwindigkeiten moduliert. **In** einem Knöchelgelenk sollte der Widerstand gegen eine Dorsalflexion in der Standphase eines Seitwärtsgehens nicht reduziert werden, im Gegensatz zum geradeaus Gehen, insbesondere in der mittleren Standphase. Auch ist die Einleitung einer Schwungphase bei passiven Prothesenkniegelenken problematisch, da bei Seitwärtsschritten eine Einleitung der Hüftbewegung nur gering ausfällt und daher nur wenig Knieflexion erzeugt werden kann. Beim geradeaus Gehen erfolgt eine Knieflexion durch die distale Masse unterhalb des Prothesenkniegelenkes und ein Aufschwingen durch die Axialentlastung und Flexion. Wird das Kniegelenk in der Standphase eingebeugt oder in der entlasteten Phase eine Einbeugung bewirkt, kann diese gebeugte Position während des Heranziehen oder des Abduzierens gehalten werden. Dies kann mit einer Erhöhung eines Extensionswiderstandes geschehen. Die Beibehaltung der flektierten Stellung ist sinnvoll, wenn es sich um eine entlastete Seitwärtsbewegung handelt, wie sie beispielsweise in der Figur 4 in der Position D und in der Figur 3 in Position A gezeigt ist. Vorteilhafterweise wird im Verlauf der Schwungphase eine Knieextension wieder freigegeben, insbesondere so rechtzeitig freigegeben bzw. gesteuert, dass das Bein in gestreckter Stellung wieder aufsetzen kann. Dies kann z.B. auf der Basis einer Erkennung der Seitwärtsbewegung, einer Erkennung einer Abbremsung einer Seitwärtsbewegung, einer Bewegungsumkehr, einer Oberschenkelbewegung und/oder zeitgesteuert geschehen.

Während bei Prothesen die gesamte Steuerung der Bewegung über den Aktuator auf der Grundlage der Sensordaten und der Auswertung in der Steuerungseinrichtung erfolgen muss, erfolgt eine solche Steuerung bei einer orthetischen Versorgung, beispielsweise einer KAFO, zumindest teilweise noch über die muskuläre Restfunktionalität. Häufig kann das versorgte Bein gegen die Schwerkraft durch die eigene Muskulatur angehoben werden. Alternativ oder ergänzend kann das Knie gebeugt und/oder der Fuß angehoben werden. Wird dann ein Seitwärtsschritt am Ende einer terminalen Standphase erkannt oder wird dies erkennbar, beispielsweise durch die Abnahme der Rotationsgeschwindigkeit oder einem entsprechenden Belastungsverlauf, ist es möglich die Widerstände, insbesondere Flexionswiderstände, in dem Kniegelenk zu reduzieren und es dem Nutzer dadurch zu ermöglichen, die Orthese mit einem minimalen Kraftaufwand zu bewegen. Sofern eine orthetische Versorgung auch die Hüfte und das Hüftgelenk einschließt, ist bei einer Seitwärtsbewegung auch eine Hüftflexion und -extension freigebbar.

Bei aktiven orthopädietechnischen Einrichtungen 100 können die Freiheitsgrade bei Seitwärtsschritten aktiv angesteuert werden. Ein Prothesenkniegelenk oder ein Orthesenkniegelenk kann in der Entlastungsphase und der Schwungphase, also bei abnehmender Axialbelastung oder nicht mehr vorhandener Axialbelastung, eingebeugt werden, um eine ausreichende Bodenfreiheit zu erzeugen und um Kompensationsbewegungen durch Plantarflexion der kontralateralen Seite zu reduzieren, auch "Vaulting" genannt. Der Kniewinkel kann in der Schwungphase so eingestellt bzw. begrenzt werden, dass sich der Abstand zwischen der Hüfte und dem Fuß um einen festgelegten oder relativen Betrag verkürzt. Der Abstand zwischen dem Hüftgelenk und dem Knöchelgelenk ist die sogenannte Beinsehne, die zur Steuerung auch eines Seitwärtsschrittes bei einer Neigung innerhalb der Frontalebene herangezogen werden kann. Die Länge der Beinsehne ist dabei eine wesentliche Steuergröße. Durch eine Anhebung der Fußspitze oder Dorsalflexion kann die Bodenfreiheit ebenfalls beeinflusst werden, wobei das Beibehalten des Fußes in der Neutralposition ausreichen kann. Auch die Orientierung der Beinsehne kann für die Steuerung herangezogen werden, insbesondere deren Komponenten in der Frontalebene.

Der Kniewinkel kann so eingestellt oder gesteuert werden, dass der Fuß ungefähr unterhalb des Hüftgelenkes bleibt, sodass sich der Kniebeugewinkel erhöht, wenn eine stärkere Hüftbeugung durchgeführt wird. Der maximale Beugewinkel wird vorteilhafterweise begrenzt, sodass bei einer Aufsetzbewegung des Fußes die Streckbewegung rechtzeitig erfolgen kann. Die Streckbewegung innerhalb des Kniewinkels oder ein Ende einer aktiven Einbeugung kann erfolgen, indem die Bewegung des Oberschenkels erfasst wird. Wird eine Hüftextension detektiert, wird eine Extension erleichtert oder initiiert.

Sofern ein entsprechender Antrieb oder eine Aktivierung möglich ist, kann ein Hüftgelenk aktiviert werden, um ein Anheben des Fußes zu erzielen.

Bei einem aktiv angetriebenen Fuß kann in der Standphase einer Seitwärtsbewegung, bei der der Körperschwerpunkt zunächst von der versorgten, aufgesetzten Seite auf die kontralaterale Seite verlagert und die kontralaterale Seite dann aufgesetzt wird, eine Plantarflexion ausgeführt werden, um die Seitwärtsbewegung zu unterstützen. Auf diese Art und Weise wird verhindert, dass der Körperschwerpunkt während einer Seitwärtsbewegung und einer Neigung innerhalb der Frontalebene absinkt, da die effektive Beinlänge der versorgten Seite vergrößert wird. Der Übergang zwischen einer solchen quasi-statischen Längenkompensation und einem aktiven Abdrücken ist fließend und ist von der Dynamik der Bewegung abhängig.

Wird die versorgte Seite mit der orthopädietechnischen Einrichtung in der Schwungphase abduziert, also in Lateralrichtung bewegt und zur Seite gestellt, kann es vorteilhaft sein, den Fuß in der Absetzphase plantar zu flektieren und ein kontrolliertes Aufsetzen auf dem Vorfuß zu ermöglichen. Als Zielgröße ist dabei die funktionell vertikale Beinlänge oder Bodenfreiheit vorgebbar, die bei zunehmender Abduktion konstant bleibt oder konstant gehalten werden soll. Alternativ oder ergänzend kann ein transienter Verlauf vorgegeben werden, insbesondere ein zeitlich vorgegebener Verlauf. Findet gleichzeitig ein Schritt nach vorne statt, sollte es zu keiner Plantarflexion kommen, damit die Vorwärtsbewegung durch einen Fersenstoß und ein Abrollen über den gesamten Fuß erleichtert wird. Bei einem Rückwärtsschritt ist eine Plantarflexion vorteilhaft.

Moderne prothetische Einrichtungen weisen Sondermodi auf, beispielsweise für Fahrrad fahren, Sitzen, Rudern oder andere besondere Bewegungen, insbesondere repetitive Bewegungen. Wenn eine Seitwärtsneigung oder eine Seitwärtsverschwenkung erkannt wird, kann die Erkennung einer Seitwärtsneigung oder plötzlichen Abduktion dazu genutzt werden, einen entsprechenden Sondermodus abzuschalten und einen Standardmodus oder einen Sicherheitsmodus zu aktivieren, um eine maximale Sicherheit für den Nutzer der orthopädietechnischen Einrichtung bereitzustellen.

Es ist auch möglich eine Verschwenkung in der Frontalebene in Situationen für die Steuerung heranzuziehen, die nicht einer Gehsituation entsprechen. Zum Beispiel kann ein Stehen in leicht oder stark gegrätschter Position erkannt und die Steuerung gegenüber dem Stehen in mehr geschlossener Fußstellung und/oder vertikaler Beinorientierung angepasst werden. Ein Fuß mit einem Freiheitsgrad in Inversions-Eversions-Freiheitsgraden kann beispielsweise so gesteuert werden, dass die Fußsohle plan auf dem Boden aufliegt, anstatt auf der Innenkante.

Ebenfalls kann eine Seitwärtsneigung und/oder Bewegung in einer Sonderfunktion herangezogen werden. Zum Beispiel kann beim Skifahren die Seitwärtsneigung bestimmt werden und die Steuerung entsprechend angepasst werden. Insbesondere kann über die Seitwärtsneigung zwischen Berg- und Talski unterschieden werden. Bei einer Neigung nach lateral ist die entsprechende Seite der Bergski, bei einer Neigung nach medial der Talski. Um zwischen einer Neigung nach medial und lateral zu unterscheiden, ist es notwendig zu wissen, ob es sich um ein linkes oder rechtes Bein handelt. Eine solche Information kann in der orthopädietechnischen Einrichtung hinterlegt sein oder aus Sensorwerten berechnet werden. Stellt die versorgte Seite den Talski dar, ist es zum Beispiel möglich, eine vollständige Streckung eines Kniegelenks zu unterbinden, zum Beispiel durch Anhebung des Bewegungswiderstands in Extensionsrichtung. Dadurch kann mehr Druck auf den vorderen Teil des Skis ausgeübt werden. Nimmt die versorgte Seite die Bergski-Seite ein, kann die Streckung wieder vollständig zugelassen werden. Über die Verkippung kann der Wechsel zwischen Berg- und Talski erkannt werden und der Übergang ggf. fließend gestaltet werden. Neben dem Skifahren kann auch in anderen Sonderfunktionen die Steuerung in Abhängigkeit der Seitwärtsneigung und/oder Bewegung angepasst werden, insbesondere Bewegungswiderstände und/oder Bewegungen angepasst werden.

Eine Orthese oder ein Exoskelett kann eine Bewegung zulassen, ihr entgegenwirken oder eine Bewegung unterstützen. Bei einem Exoskelett weist die nutzende Person zumeist keine wesentlichen Einschränkungen des Bewegungsapparates auf. Entsprechend werden Bewegungen unterstützt, um beispielsweise die Belastung des Körpers zu reduzieren, die Leistungsfähigkeit zu steigern oder den Komfort zu erhöhen. Bei einer Orthese wird zumeist eine Insuffizienz der nutzenden Person kompensiert, bspw. muskuläre oder neuronale Insuffizienz durch entsprechende Unterstützung kompensiert. Die Unterstützung durch ein Exoskelett und/oder einer Orthese kann bei einer Seitwärtsbewegung angepasst werden. Es kann zum Beispiel eine Bewegung zugelassen werden, wenn eine Seitwärtsbewegung und/oder Neigung detektiert wird. Es ist auch möglich, dass eine Bewegung, die durch die nutzende Person initiiert wird, durch die Orthese oder das Exoskelett erleichtert wird, das heißt, dass die notwendigen Kräfte und Momente, die durch den Körper aufgebracht werden müssen, um eine Bewegung durchzuführen, verringert werden. Bei einem Seitwärtsschritt ist es zum Beispiel möglich, dass das Gewicht der Orthese oder des Exoskeletts und/oder das Gewicht der eigenen Gliedmaße übernommen werden. Es werden also Kräfte und Momente aufgebracht, welche der Schwerkraft entgegenwirken. **In** einer Entlastungs- und/oder Anhebephase wird ein kniebeugendes und/oder hüftflektierendes Moment aufgebracht, um das Abwinkeln des Beines zu erleichtern. Während der Schwungphase kann durch ein kniebeugendes und/oder hüftflektierendes Moment das Bein in abgewinkelter Stellung gehalten werden. Durch ein hüftabduzierendes oder hüftadduzierendes Moment kann das zur Seite Schwingen des Beines, respektive das seitliche Positionieren des Fußes, erleichtert werden. Durch ein plantar flektierendes Moment kann in einer Entlastungsphase das Einbeugen des Beines erleichtert werden. Ein dorsalextendierendes Moment in der Anhebephase, aber auch in der Schwungphase während der Positionierung des Fußes, kann das Anheben des Fußes erleichtern und somit eine ausreichende Bodenfreiheit in der Schwungphase erzielen. **In** einer Absetzphase kann das hüftbeugende, kniebeugende und oder dorsalextendierende Moment reduziert werden, um eine Streckung des Beines sowie ein kontrolliertes Absetzen des Fußes zu ermöglichen. Alternativ oder ergänzend kann auch ein hüftextendierendes, kniestreckendes und/oder plantar flektierendes Moment aufgebracht werden, um das Absenken aktiv zu unterstützen. **In** der Standphase kann auch ein Hüftabduktionsmoment und/oder Hüftadduktionsmoment aufgebracht werden, um das Seitwärtsgehen zu unterstützen.

Bei der Nutzung eines Exoskeletts ist es möglich, das Gewicht sowie die Trägheitseffekte des Exoskeletts selber zu kompensieren oder zu reduzieren. Durch Aufbringen von Kräften und Momenten, die diesen Gewichts- und/oder Trägheitseffekten entgegenwirken, kann der Tragekomfort erhöht und der Aufwand der nutzenden Person für die Durchführung von Bewegungen reduziert werden. Idealerweise erscheint das Exoskelett aus Sicht der nutzenden Person mechanisch transparent, das heißt, das zusätzliche Gewicht und/oder die Trägheit sind nicht spürbar. Für eine solche Steuerung ist es möglich, Bewegungen und Bewegungsänderungen in der Frontalebene zu erfassen und mit einzubeziehen. Insbesondere eine Verkippung in der Frontalebene gegenüber der Schwerkraft oder einem Potentialfeld oder aber auch die rotatorische Bewegung in der Frontalebene können für die Steuerung herangezogen werden. Wird zum Beispiel bei einem knieübergreifenden Exoskelett ein entlastetes, abgewinkeltes Bein, bei dem sich der Fuß unter der Hüfte befindet, in Relation zur Schwerkraft seitlich verkippt, das heißt in der Frontalebene von der Vertikalen in Richtung der Horizontalen verkippt, reduziert sich das kniestreckende Moment aufgrund des Gewichts des Unterschenkelsegments des Exoskeletts. Wird in vertikaler Position durch einen Aktuator des Exoskeletts ein kniebeugendes Moment aufgebracht, um der kniestreckenden Wirkung des Unterschenkelgewichts entgegenzuwirken, kann dieses Moment mit zunehmender Seitwärtsneigung reduziert werden. Gleiches gilt für eine Orthese.

## Patentansprüche

1. Verfahren zur Steuerung einer orthopädietechnischen Einrichtung (100) der unteren Extremität mit einem Oberteil (2) und einem Unterteil (3), die gelenkig um zumindest eine Schwenkachse (4) miteinander unter Ausbildung eines Gelenkes (5) aneinander gelagert sind und zumindest einem Aktuator (6), der mit einer Steuerungseinrichtung (7) gekoppelt ist, die auf der Grundlage von Sensordaten zumindest eines mit der Steuerungseinrichtung (7) gekoppelten Sensors (8) den Aktuator (6) aktiviert oder deaktiviert, um einen Verschwenkungswiderstand und/oder eine Relativbewegung des Oberteils (2) zu dem Unterteil (3) zu beeinflussen, wobei über die Sensordaten eine Orientierung und/oder Verlagerung der orthopädietechnischen Einrichtung (100) in der Frontalebene erfasst wird und auf der Grundlage der Orientierung und/oder Verlagerung in der Frontalebene der Aktuator (6) aktiviert, deaktiviert oder ein Sollwert für den Aktuator (6) moduliert wird, **dadurch gekennzeichnet, dass** in Abhängigkeit von der Neigung oder Verschwenkung in der Frontalebene der maximale Verschwenkwinkel des Oberteils (2) zu dem Unterteil (3) verstellt wird.

2. Verfahren nach Anspruch 1, **dadurch gekennzeichnet, dass** während der Nutzung der orthopädietechnischen Einrichtung (100) im angelegten Zustand die Sensordaten ermittelt werden und der Aktuator (6) aktiviert, deaktiviert oder der Sollwert für den Aktuator (6) moduliert wird.

3. Verfahren nach Anspruch 1 oder 2, **dadurch gekennzeichnet, dass** die Orientierung oder Verlagerung über einen Raumlagesensor, eine IMU und/oder Winkelsensoren erfasst und ermittelt werden.

4. Verfahren nach einem der voranstehenden Ansprüche, **dadurch gekennzeichnet, dass** Kräfte, Momente und/oder Beschleunigungen über Sensoren (8) erfasst und der Steuerung zugrundegelegt werden.

5. Verfahren nach einem der voranstehenden Ansprüche, **dadurch gekennzeichnet, dass** die orthopädietechnische Einrichtung (100) als Prothese oder Orthese ausgebildet ist und ein künstliches Kniegelenk (5) und/oder ein künstliches Knöchelgelenk (5) aufweist, denen der Aktuator (6) zugeordnet ist.

6. Verfahren nach einem der voranstehenden Ansprüche, **dadurch gekennzeichnet, dass** translatorische Verlagerungen der orthopädietechnischen Einrichtung (100) erfasst und der Steuerung zugrundegelegt werden.

7. Verfahren nach einem der voranstehenden Ansprüche, **dadurch gekennzeichnet, dass** bei einer Neigung oder Verschwenkung der versorgten Seite in der Standphase in Medialrichtung eine Verringerung eines Flexionswiderstandes oder eine Flexion bei Erreichen eines Schwellwertes der Neigung eingeleitet wird.

8. Verfahren nach Anspruch 7, **dadurch gekennzeichnet, dass** bei einer Verringerung der Axialbelastung oder in einer Anhebephase der versorgten Seite eine Verringerung des Flexionswiderstandes oder eine Flexion eingeleitet wird.

9. Verfahren nach Anspruch 7 oder 8, **dadurch gekennzeichnet, dass** eine Verringerung eines Flexionswiderstandes oder eine Flexion nur bei einer Vorwärtsneigung oder Vorwärtsverschwenkung eingeleitet wird.

10. Verfahren nach einem der Ansprüche 1 bis 6, **dadurch gekennzeichnet, dass** bei einer Neigung oder Verschwenkung der versorgten Seite in der Standphase in Medialrichtung bei gleichzeitiger Erfassung einer Rückwärtsneigung oder Rückwärtsverschwenkung keine Verringerung oder eine Erhöhung eines Flexionswiderstandes eingeleitet wird.

11. Verfahren nach einem der Ansprüche 1 bis 6, **dadurch gekennzeichnet, dass** bei einer Neigung oder Verschwenkung der versorgten Seite in der Standphase in Medialrichtung bei gleichzeitiger Erfassung einer Rückwärtsneigung oder Rückwärtsverschwenkung eine Flexionsbewegung abgestoppt oder eine Extensionsbewegung eingeleitet wird.

12. Verfahren nach einem der voranstehenden Ansprüche, **dadurch gekennzeichnet, dass** bei einer Neigung oder Verschwenkung der versorgten Seite in der Standphase in Medialrichtung ein künstliches Knöchelgelenk (5) in Plantarflexionsrichtung angetrieben oder der Dorsalflexionswiderstand erhöht oder nicht verringert wird.

13. Verfahren nach einem der voranstehenden Ansprüche, **dadurch gekennzeichnet, dass** bei einer Neigung oder Verschwenkung der versorgten Seite in der Standphase und einem festgestellten Flexionsmoment keine Verringerung oder eine Erhöhung eines Flexionswiderstandes eingeleitet wird oder ein Extensionsmoment aufgebracht wird.

14. Verfahren nach einem der voranstehenden Ansprüche, **dadurch gekennzeichnet, dass** bei einer Verschwenkung der versorgten Seite in einer Anhebephase der Schwungphase in Lateralrichtung eine Verringerung eines Flexionswiderstandes oder eine Flexion eingeleitet wird.

15. Verfahren nach Anspruch 14, **dadurch gekennzeichnet, dass** eine Extension in einem künstlichen Kniegelenk (5) in der Schwungphase verzögert oder verhindert wird.

16. Verfahren nach Anspruch 15, **dadurch gekennzeichnet, dass** eine Extension in einem künstlichen Kniegelenk (5) zeitgesteuert, nach Erreichen eines Verschwenkwinkels, einer Veränderung der Verschwenkgeschwindigkeit, einer Veränderung der Verschwenkrichtung oder auf der Grundlage einer Hüftextensionsbewegung freigegeben oder bewirkt wird.

17. Verfahren nach einem der voranstehenden Ansprüche, **dadurch gekennzeichnet, dass** auf der Grundlage der Neigung oder Verlagerung in der Frontalebene ein Sondermodus der Steuerung aktiviert oder deaktiviert wird.

18. Orthopädietechnische Einrichtung (100) der unteren Extremität mit einem Oberteil (2) und einem Unterteil (3), die gelenkig um zumindest eine Schwenkachse (4) miteinander unter Ausbildung eines Gelenkes (5) aneinander gelagert sind und zumindest einem Aktuator (6), der mit einer Steuerungseinrichtung (7) gekoppelt ist, die auf der Grundlage von Sensordaten zumindest eines mit der Steuerungseinrichtung (7) gekoppelten Sensors (8) den Aktuator (6) aktiviert oder deaktiviert, um einen Verschwenkungswiderstand und/oder eine Relativbewegung des Oberteils (2) zu dem Unterteil (3) zu beeinflussen, **dadurch gekennzeichnet, dass** der zumindest eine Sensor (8) ausgebildet und eingerichtet ist, Sensordaten über eine Orientierung und/oder Verlagerung der orthopädietechnischen Einrichtung (100) in der Frontalebene zu erfassen und dass die Steuerungseinrichtung (7) eingerichtet ist, auf der Grundlage der Orientierung und/oder Verlagerung in der Frontalebene den Aktuator (6) zu aktivieren, zu deaktivieren oder einen Sollwert für den Aktuator (6) zu modulieren und dass in Abhängigkeit von der Neigung oder Verschwenkung in der Frontalebene der maximale Verschwenkwinkel des Oberteils (2) zu dem Unterteil (3) verstellt wird.

19. Orthopädietechnische Einrichtung (100) nach Anspruch 18, **dadurch gekennzeichnet, dass** der zumindest eine Sensor (7) als IMU ausgebildet ist und an dem Oberteil (2) oder dem Unterteil (3) befestigt ist.

20. Orthopädietechnische Einrichtung (100) nach Anspruch 18 oder 19, **dadurch gekennzeichnet, dass** zumindest ein Kraftsensor, Beschleunigungssensor, Winkelsensor und/oder Momentensensor an dem Oberteil (2) und/oder dem Unterteil (3) angeordnet ist.

## Claims

1. A method for controlling an orthopedic device (100) of the lower extremity having an upper part (2) and a lower part (3), which are mounted on one another in an articulated manner around at least one pivot axis (4) to form a joint (5). and at least one actuator (6), which is coupled to a control unit (7), which activates or deactivates the actuator (6) on the basis of sensor data of at least one sensor (8) coupled with the control unit (7) in order to influence a pivot resistance and/or a relative movement of the upper part (2) in relation to the lower part (3), wherein an orientation and/or displacement of the orthopedic device (100) in the frontal plane is detected via the sensor data and the actuator (6) is activated or deactivated or a target value for the actuator (6) is modulated on the basis of the orientation and/or displacement in the frontal plane, **characterized in that** the maximum pivot angle of the upper part (2) in relation to the lower part (3) is adjusted depending on the inclination or pivot in the frontal plane.

2. The method as claimed in claim 1, **characterized in that**, during the use of the orthopedic device (100) in the applied state, the sensor data are determined and actuator (6) is activated or deactivated or the target value for the actuator (6) is modulated.

3. The method as claimed in claim 1 or 2, **characterized in that** the orientation or displacement are detected and determined via a spatial position sensor, an IMU, and/or angle sensors.

4. The method as claimed in any one of the preceding claims, **characterized in that** forces, torques, and/or accelerations are detected via sensors (8) and used as the basis for the control.

5. The method as claimed in any one of the preceding claims, **characterized in that** the orthopedic device (100) is designed as a prosthesis or orthosis and has an artificial knee joint (5) and/or an artificial ankle joint (5), to which the actuator (6) is assigned.

6. The method as claimed in any one of the preceding claims, **characterized in that** translational displacements of the orthopedic device (100) are detected and used as the basis for the control.

7. The method as claimed in any one of the preceding claims, **characterized in that**, upon inclination or pivot of the treated side in the standing phase in the medial direction, a reduction of the flexion resistance or a flexion is initiated upon reaching a threshold value of the inclination.

8. The method as claimed in claim 7, **characterized in that**, upon a reduction of the axial load or in a lifting phase of the treated side, a reduction of the flexion resistance or a flexion is initiated.

9. The method as claimed in claim 7 or 8, **characterized in that** a reduction of a flexion resistance or a flexion is only initiated during a forward inclination or forward pivot.

10. The method as claimed in any one of claims 1 to 6, **characterized in that**, upon an inclination or pivot of the treated side in the standing phase in the medial direction with simultaneous detection of a backward inclination or backward pivot, no reduction or an increase of a flexion resistance is initiated.

11. The method as claimed in any one of claims 1 to 6, **characterized in that**, upon an inclination or pivot of the treated side in the standing phase in the medial direction with simultaneous detection of a backward inclination or backward pivot, a flexion movement is stopped or an extension movement is initiated.

12. The method as claimed in any one of the preceding claims, **characterized in that**, upon an inclination or pivot of the treated side in the standing phase in the medial direction, an artificial ankle joint (5) is driven in the plantar flexion direction or the dorsiflexion resistance is increased or not reduced.

13. The method as claimed in any one of the preceding claims, **characterized in that**, upon an inclination or pivot of the treated side in the standing phase and an established flexion torque, no reduction or an increase of a flexion resistance is initiated or an extension torque is applied.

14. The method as claimed in any one of the preceding claims, **characterized in that**, upon a pivot of the treated side in a lifting phase of the swinging phase in the lateral direction, a reduction of a flexion resistance or a flexion is initiated.

15. The method as claimed in claim 15, **characterized in that** an extension in an artificial knee joint (5) is delayed or prevented in the swinging phase.

16. The method as claimed in claim 15, **characterized in that** an extension in an artificial knee joint (5) is released or caused in a time-controlled manner, after reaching a pivot angle, a change of the pivot speed, a change of the pivot direction, or on the basis of a hip extension movement.

17. The method as claimed in any one of the preceding claims, **characterized in that** a special mode of the control is activated or deactivated on the basis of the inclination or displacement in the frontal plane.

18. An orthopedic device (100) of the lower extremity having an upper part (2) and a lower part (3), which are mounted with one another in an articulated manner around at least one pivot axis (4) to form a joint (5), and at least one actuator (6), which is coupled to a control unit (7), which activates or deactivates the actuator (6) 2on the basis of sensor data of at least one sensor (8) coupled to the control unit (7) in order to influence a pivot resistance and/or a relative movement of the upper part (2) in relation to the lower part (3), **characterized in that** the at least one sensor (8) is designed and configured to detect sensor data about an orientation and/or displacement of the orthopedic device (100) in the frontal plane and that the control unit (7) is configured to activate or deactivate the actuator (6) or modulate a target value for the actuator (6) on the basis of the orientation and/or displacement in the frontal plane and **in that** the maximum pivot angle of the upper part (2) in relation to the lower part (3) is adjusted depending on the inclination or pivot in the frontal plane.

19. The orthopedic device (100) as claimed in claim 18, **characterized in that** the at least one sensor (7) is designed as an IMU and is fastened on the upper part (2) or the lower part (3).

20. The orthopedic device (100) as claimed in claim 18 or 19, **characterized in that** at least one force sensor, acceleration sensor, angle sensor, and/or torque sensor is arranged on the upper part (2) and/or the lower part (3).

## Revendications

1. Procédé de commande d'un dispositif orthopédique (100) destiné aux membres inférieurs, comprenant une partie supérieure (2) et une partie inférieure (3) qui sont articulées l'une à l'autre autour d'au moins un axe de pivotement (4) en formant une articulation (5), et au moins un actionneur (6) couplé à un dispositif de commande (7) qui, en se basant sur des données de capteur fournies par au moins un capteur (8) couplé au dispositif de commande (7), vient activer ou désactiver l'actionneur (6) afin d'influencer une résistance au pivotement et/ou un mouvement relatif de la partie supérieure (2) par rapport à la partie inférieure (3), les données de capteur permettant de détecter une orientation et/ou un déplacement du dispositif orthopédique (100) dans le plan frontal et, sur la base de l'orientation et/ou du déplacement dans le plan frontal, l'actionneur (6) est activé ou désactivé ou une valeur de consigne pour l'actionneur (6) est modulée,
**caractérisé en ce que**, en fonction de l'inclinaison ou du pivotement dans le plan frontal, l'angle de pivotement maximal de la partie supérieure (2) par rapport à la partie inférieure (3) est ajusté.

2. Procédé selon la revendication 1,
**caractérisé en ce que**, pendant l'utilisation du dispositif orthopédique (100) à l'état appliqué, les données de capteur sont déterminées et l'actionneur (6) est activé ou désactivé ou la valeur de consigne pour l'actionneur (6) est modulée.

3. Procédé selon la revendication 1 ou 2,
**caractérisé en ce que** l'orientation ou le déplacement sont détectés et déterminés à l'aide d'un capteur de position spatiale, d'une centrale à inertie (IMU) et/ou de capteurs d'angle.

4. Procédé selon l'une des revendications précédentes,
**caractérisé en ce que** les forces, les couples et/ou les accélérations sont détectés à l'aide de capteurs (8) et servent de base à la commande.

5. Procédé selon l'une des revendications précédentes,
**caractérisé en ce que** le dispositif orthopédique (100) est conçu sous la forme d'une prothèse ou d'une orthèse et comporte une articulation artificielle de genou (5) et/ou une articulation artificielle de cheville (5), auxquelles est associé l'actionneur (6).

6. Procédé selon l'une des revendications précédentes,
**caractérisé en ce que** les déplacements en translation du dispositif orthopédique (100) sont détectés et servent de base à la commande.

7. Procédé selon l'une des revendications précédentes,
**caractérisé en ce que**, lors d'une inclinaison ou d'un pivotement du côté traité dans la direction médiale pendant la phase d'appui, une diminution de la résistance à la flexion ou une flexion est déclenchée lorsqu'une valeur seuil de l'inclinaison est atteinte.

8. Procédé selon la revendication 7,
**caractérisé en ce que**, lors d'une diminution de la charge axiale ou pendant une phase de levée du côté traité, une diminution de la résistance à la flexion ou une flexion est déclenchée.

9. Procédé selon la revendication 7 ou 8,
**caractérisé en ce qu'**une diminution de la résistance à la flexion ou une flexion n'est déclenchée qu'en cas d'inclinaison vers l'avant ou de pivotement vers l'avant.

10. Procédé selon l'une des revendications 1 à 6,
**caractérisé en ce que**, lors d'une inclinaison ou d'un pivotement du côté traité dans la direction médiale pendant la phase d'appui, avec détection simultanée d'une inclinaison vers l'arrière ou d'un pivotement vers l'arrière, aucune diminution ni augmentation de la résistance à la flexion n'est déclenchée.

11. Procédé selon l'une des revendications 1 à 6,
**caractérisé en ce que**, lors d'une inclinaison ou d'un pivotement du côté traité dans la direction médiale pendant la phase d'appui, avec détection simultanée d'une inclinaison vers l'arrière ou d'un pivotement vers l'arrière, un mouvement de flexion est arrêté ou un mouvement d'extension est déclenché.

12. Procédé selon l'une des revendications précédentes,
**caractérisé en ce que**, lors d'une inclinaison ou d'un pivotement du côté traité dans la direction médiale pendant la phase d'appui, une articulation artificielle de cheville (5) est entraînée dans le sens de la flexion plantaire, ou la résistance à la flexion dorsale est augmentée ou n'est pas réduite.

13. Procédé selon l'une des revendications précédentes,
**caractérisé en ce que**, lors d'une inclinaison ou d'un pivotement du côté traité pendant la phase d'appui et lorsqu'un couple de flexion est détecté, aucune diminution ni augmentation de la résistance à la flexion n'est déclenchée, ou aucun couple d'extension n'est appliqué.

14. Procédé selon l'une des revendications précédentes,
**caractérisé en ce que**, lors d'un pivotement du côté traité dans la direction latérale pendant une phase de levée de la phase pendulaire, une diminution de la résistance à la flexion ou une flexion est déclenchée.

15. Procédé selon la revendication 14,
**caractérisé en ce qu'**une extension dans une articulation artificielle de genou (5) pendant la phase pendulaire est retardée ou empêchée.

16. Procédé selon la revendication 15,
**caractérisé en ce qu'**une extension dans une articulation artificielle de genou (5) est autorisée ou provoquée de manière temporisée après obtention d'un angle de pivotement, d'une modification de la vitesse de pivotement, d'une modification de la direction de pivotement ou sur la base d'un mouvement d'extension de la hanche.

17. Procédé selon l'une des revendications précédentes,
**caractérisé en ce qu'**un mode spécial de commande est activé ou désactivé sur la base de l'inclinaison ou du déplacement dans le plan frontal.

18. Dispositif orthopédique (100) destiné aux membres inférieurs, comprenant une partie supérieure (2) et une partie inférieure (3) qui sont articulées l'une à l'autre autour d'au moins un axe de pivotement (4) en formant une articulation (5), et au moins un actionneur (6) couplé à un dispositif de commande (7) qui, en se basant sur des données de capteur fournies par au moins un capteur (8) couplé au dispositif de commande (7), vient activer ou désactiver l'actionneur (6) afin d'influencer une résistance au pivotement et/ou un mouvement relatif de la partie supérieure (2) par rapport à la partie inférieure (3),
**caractérisé en ce que** ledit au moins un capteur (8) est conçu et agencé pour acquérir des données de capteur concernant une orientation et/ou un déplacement du dispositif orthopédique (100) dans le plan frontal, et **en ce que** le dispositif de commande (7) est conçu pour, sur la base de l'orientation et/ou du déplacement dans le plan frontal, activer ou désactiver l'actionneur (5) ou pour moduler une valeur de consigne pour l'actionneur (6), et **en ce que**, en fonction de l'inclinaison ou du pivotement dans le plan frontal, l'angle de pivotement maximal de la partie supérieure (2) par rapport à la partie inférieure (3) est ajusté.

19. Dispositif orthopédique (100) selon la revendication 18,
**caractérisé en ce que** ledit au moins un capteur (7) est conçu comme une centrale à inertie (IMU) et est fixé à la partie supérieure (2) ou à la partie inférieure (3).

20. Dispositif orthopédique (100) selon la revendication 18 ou 19,
**caractérisé en ce qu'**au moins un capteur de force, un capteur d'accélération, un capteur d'angle et/ou un capteur de couple est disposé sur la partie supérieure (2) et/ou sur la partie inférieure (3).
